(19) Europäisches Patentamt
European Patent Office
Office européen des brevets

(11) EP 2 148 295 A1

(12) EUROPEAN PATENT APPLICATION
published in accordance with Art. 153(4) EPC

(43) Date of publication:
**27.01.2010 Bulletin 2010/04**

(21) Application number: **08740827.4**

(22) Date of filing: **24.04.2008**

(51) Int Cl.:
***G06T 1/00*** *(2006.01)* ***A61B 5/117*** *(2006.01)*
***G06T 7/00*** *(2006.01)*

(86) International application number:
**PCT/JP2008/057950**

(87) International publication number:
**WO 2008/139883 (20.11.2008 Gazette 2008/47)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **16.05.2007 JP 2007130801**

(71) Applicant: **Sony Corporation**
**Tokyo 108-0075 (JP)**

(72) Inventor: **ABE, Hiroshi**
**Tokyo 180-0075 (JP)**

(74) Representative: **Robinson, Nigel Alexander Julian**
**D Young & Co**
**120 Holborn**
**London EC1N 2DY (GB)**

# (54) VEIN PATTERN MANAGEMENT SYSTEM, VEIN PATTERN REGISTRATION DEVICE, VEIN PATTERN AUTHENTICATION DEVICE, VEIN PATTERN REGISTRATION METHOD, VEIN PATTERN AUTHENTICATION METHOD, PROGRAM, AND VEIN DATA STRUCTURE

(57) There are provided a vein pattern management system, a vein pattern registration apparatus, a vein pattern authentication apparatus, a vein pattern registration method, a vein pattern authentication method, a program, and a vein data configuration that can determine presence of a pseudo-vein pattern intentionally formed on a body surface.

An imaging unit capturing images of the body surface of a portion of a living body with near-infrared light and visible light and generating near-infrared light imaging data and visible light imaging data, respectively, a vein pattern extraction unit extracting vein patterns from the near-infrared light imaging data and the visible light imaging data to generate a near-infrared light vein pattern and a visible light vein pattern, respectively, and a pseudo-vein pattern determination unit determining presence of a pseudo-vein pattern intentionally formed on a part of the captured body surface by comparing the near-infrared light vein pattern and the visible light vein pattern are provided.

FIG. 3

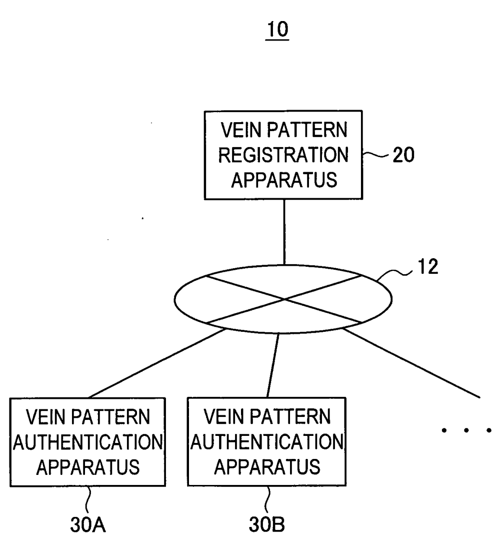




Printed by Jouve, 75001 PARIS (FR)

## Description

TECHNICAL FIELD

The present invention relates to a vein pattern management system, a vein pattern registration apparatus, a vein pattern authentication apparatus, a vein pattern registration method, a vein pattern authentication method, a program, and a vein data configuration.

BACKGROUND ART

Individual authentication methods include a method for authenticating an individual by registering a fingerprint, a voiceprint, an iris, and a retina of the individual, or a vein pattern of the back of the individual's hand or the individual's finger, or the like as registered data in advance, and verifying and determining data input at the time of authentication and the registered data. In particular, individual authentication using the vein pattern has recently been focused on due to its high discriminating ability.

For the purpose of improving security of the above-mentioned individual authentication methods, since it is essential to block illegal users attempting to impersonate normal authenticated users, methods for blocking such illegal users have been widely developed (for example, refer to Patent Document 1 and Non-Patent Document 1).

[Patent Document 1] Japanese Patent Application Publication No. 2005-259345
[Non-Patent Document 1] Tsutomu Matsumoto, "Biometric Authentication in Financial Transactions", the 9th Study Group on Problem of Forged ATM Cards", Financial Services Agency, April 15, 2005

DISCLOSURE OF THE INVENTION

PROBLEMS TO BE SOLVED BY THE INVENTION

In some individual authentication methods using a vein pattern, the vein pattern is extracted by capturing an image of a backside or a finger of a hand with near-infrared light and processing extracted imaging data using a differential filter.

However, since the differential filter used to the imaging data captured with the near-infrared light into a vein portion and a non-vein portion is apt to output a pseudo-vein pattern, which has been drawn on a body surface with a felt-tip pen and the like, as a vein portion, there is a need for a method for determining presence of such a pseudo-vein pattern in order to avoid impersonation by an illegal user.

The present invention has been made in consideration of the above-mentioned problems, and an object of the present invention is to provide a novel and improved vein pattern management system, vein pattern registration apparatus, vein pattern authentication apparatus, vein pattern registration method, vein pattern authentication method, program, and vein data configuration, capable of determining presence of a pseudo-vein pattern intentionally produced on a body surface.

MEANS FOR SOLVING THE PROBLEMS

In order to solve the above problem, according to an embodiment of the invention, there is provided a vein pattern management system for registering and authenticating a vein pattern acquired by radiating light to a portion of a living body, including an imaging unit for capturing images of a body surface of the portion of the living body with near-infrared light and visible light, and generating near-infrared light imaging data and visible light imaging data, respectively, a vein pattern extraction unit for extracting vein patterns from the near-infrared light imaging data and the visible light imaging data to generate a near-infrared light vein pattern and a visible light vein pattern, respectively, a pseudo-vein pattern determination unit for determining presence of a pseudo-vein pattern intentionally formed on a part of the captured body surface by comparing the near-infrared light vein pattern with the visible light vein pattern, a vein pattern registration unit for registering the near-infrared light vein pattern based on a determination result from the pseudo-vein pattern determination unit to generate a registered vein pattern, and a vein pattern authentication unit for comparing a newly generated near-infrared light vein pattern with the registered vein pattern based on the determination result from the pseudo-vein pattern determination unit and authenticating the newly generated near-infrared vein pattern.

In order to solve the above problem, according to another embodiment of the invention, there is provided a vein pattern registration apparatus including an imaging unit for capturing images of a body surface of a portion of a living body with near-infrared light and visible light, and generating near-infrared light imaging data and visible light

imaging data, respectively, a vein pattern extraction unit for extracting vein patterns from the near-infrared light imaging data and the visible light imaging data to generate a near-infrared light vein pattern and a visible light vein pattern, respectively, a pseudo-vein pattern determination unit for determining presence of a pseudo-vein pattern intentionally formed on a part of the captured body surface by comparing the near-infrared light vein pattern with the visible light vein pattern, and a vein pattern registration unit for registering the near-infrared light vein pattern based on a determination result from the pseudo-vein pattern determination unit to generate a registered vein pattern.

In order to solve the above problem, according to another embodiment of the invention, there is provided a vein pattern authentication apparatus including an imaging unit for capturing images of a body surface of a portion of a living body with near-infrared light and visible light, and generating near-infrared light imaging data and visible light imaging data, respectively, a vein pattern extraction unit for extracting vein patterns from the near-infrared light imaging data and the visible light imaging data to generate a near-infrared light vein pattern and a visible light vein pattern, respectively, a pseudo-vein pattern determination unit for determining presence of a pseudo-vein pattern intentionally formed on a part of the captured body surface by comparing the near-infrared light vein pattern with the visible light vein pattern; and a vein pattern authentication unit for comparing an already registered vein pattern with the near-infrared light vein pattern and authenticating the near-infrared light vein pattern based on a determination result from the pseudo-vein pattern determination unit.

The pseudo-vein pattern determination unit may determine the presence of the pseudo-vein pattern by calculating a correlation coefficient between the near-infrared light vein pattern and the visible light vein pattern.

The pseudo-vein pattern determination unit may compare the calculated correlation coefficient with a predetermined threshold value for determination, determine that the pseudo-vein pattern is not present when the calculated correlation coefficient is less than the predetermined threshold value for determination, and determine that the pseudo-vein pattern is present when the calculated correlation coefficient is equal to or greater than the predetermined threshold value for determination.

The vein pattern extraction unit may extract the near-infrared light vein pattern and the visible light vein pattern using a differential filter, which generates a larger value at a pixel that differs largely from its surrounding pixels, for each of pixels constituting the near-infrared light imaging data and the visible light imaging data, respectively.

The differential filter may be a derivative filter or a Laplacian of Gaussian (Log) filter.

In addition, the vein pattern authentication unit may authenticate a near-infrared light vein pattern based on a registered vein pattern acquired from a vein pattern registration apparatus or may authenticate a near-infrared light vein pattern based on a registered vein pattern registered within a vein pattern authentication apparatus.

In order to solve the above-mentioned problems, according to yet another embodiment of the present invention, there is provided a vein pattern registration method for registering a vein pattern acquired by radiating light to a portion of a living body, including the steps of capturing an image of a body surface of the portion of the living body with near-infrared light and generating near-infrared light imaging data, extracting a vein pattern from the near-infrared light imaging data as a near-infrared light vein pattern; capturing an image of the body surface with visible light and generating visible light imaging data, extracting a vein pattern from the visible light imaging data as a visible light vein pattern, comparing the near-infrared light vein pattern with the visible light vein pattern and determining presence of a pseudo-vein pattern intentionally formed on a part of the body surface, and registering the near-infrared light vein pattern based on a determination result.

In order to solve the above-mentioned problems, according to yet another embodiment of the present invention, there is provided a vein pattern authentication method for authenticating a vein pattern acquired by radiating light to a portion of a living body, including the steps of capturing an image of a body surface of the portion of the living body with near-infrared light and generating a near-infrared light imaging data, extracting a vein pattern from the near-infrared light imaging data as a near-infrared light vein pattern, capturing an image of the body surface with visible light and generating visible light imaging data; extracting a vein pattern from the visible light imaging data as a visible light vein pattern, comparing the near-infrared light vein pattern with the visible light vein pattern and determining presence of a pseudo-vein pattern intentionally formed on a part of the body surface, and comparing an already registered vein pattern with the near-infrared light vein pattern and authenticating the near-infrared light vein pattern based on a determination result.

The step of comparing the near-infrared light vein pattern with the visible light vein pattern may include the step of calculating a correlation coefficient between the near-infrared light vein pattern and the visible light vein pattern.

The step of determining presence of a pseudo-vein pattern may include the steps of: comparing the calculated correlation coefficient with a predetermined threshold value for determination; determining that the pseudo-vein pattern is not present when the calculated correlation coefficient is less than the predetermined threshold value for determination; and determining that the pseudo-vein pattern is present when the calculated correlation coefficient is equal to or greater than the predetermined threshold value for determination.

The step of generating the near-infrared light vein pattern and the step of generating the visible light vein pattern may include the step of using a differential filter that outputs a large value for a pixel having a large difference between

the pixel and its surrounding pixels for a plurality of pixels constituting the near-infrared light imaging data and the visible light imaging data, respectively.

The differential filter may be a derivative filter or a Laplacian of Gaussian (Log) filter.

In order to solve the above-mentioned problems, according to yet another embodiment of the present invention, there is provided a program for causing a computer controlling a vein pattern registration apparatus for registering a vein pattern acquired by radiating light to a portion of a living body to execute an imaging function for capturing images of a body surface of the portion of the living body with near-infrared light and visible light and generating near-infrared light imaging data and visible light imaging data, respectively, a vein pattern extraction function for extracting vein patterns from the near-infrared light imaging data and the visible light imaging data as a near-infrared light vein pattern and a visible light vein pattern, respectively, a pseudo vein pattern determination function for comparing the near-infrared light vein pattern with the visible light vein pattern and determining presence of a pseudo-vein pattern intentionally formed on a part of the captured body surface, and a vein pattern registration function for registering the near-infrared light vein pattern based on a determination result to generate a registered vein pattern.

According to this configuration, a computer program is stored in a storage unit included in a computer, and read and executed by CPU included in the computer so that the computer program causes the computer to operate as the above-mentioned vein pattern registration apparatus. In addition, there can be also provided a computer readable recording medium in which the computer program is recorded. The recording medium may be, for example, a magnetic disk, an optical disk, a magnetic optical disk, a flush memory, and the like. Furthermore, the above-mentioned computer program may be distributed via a network without using a recording medium.

In order to solve the above-mentioned problems, according to yet another embodiment of the present invention, there is provided a program for causing a computer controlling a vein pattern authentication apparatus for authenticating a vein pattern acquired by radiating light to a portion of a living body to execute an imaging function for capturing images of a body surface of the portion of the living body with near-infrared light and visible light, and generating near-infrared light imaging data and visible light imaging data, respectively, a vein pattern extraction function for extracting vein patterns from the near-infrared light imaging data and the visible light imaging data to generate a near-infrared light vein pattern and a visible light vein pattern, respectively, a pseudo-vein pattern determination function for determining presence of a pseudo-vein pattern intentionally formed on a part of the captured body surface by comparing the near-infrared light vein pattern with the visible light vein pattern; and a vein pattern authentication function for comparing an already registered vein pattern with the near-infrared light vein pattern and authenticating the near-infrared light vein pattern based on a determination result.

According to this configuration, a computer program is stored in a storage unit included in a computer, and read and executed by CPU included in the computer so that the computer program causes the computer to operate as the above-mentioned vein pattern authentication apparatus. In addition, there can be also provided a computer readable recording medium in which the computer program is recorded. The recording medium may be, for example, a magnetic disk, an optical disk, a magnetic optical disk, a flush memory, and the like. Furthermore, the above-mentioned computer program may be distributed via a network without using a recording medium.

In order to solve the above-mentioned problems, according to yet another embodiment of the present invention, there is provided a vein data configuration including a vein data storage area containing data that correspond to a vein pattern of an individual and are to be verified with image data acquired by capturing an image of a body surface of a portion of a living body with near-infrared light; and a correlation coefficient storage area containing a correlation coefficient between the image data acquired by capturing the image with the near-infrared light and image data acquired by capturing an image of the body surface with visible light.

The vein data configuration further includes a parameter storage area containing a parameter changing an output property of a differential filter delivering a large output for an pixel that differs largely from its surrounding pixels, for each pixel constituting the image data acquired by capturing the image with the near-infrared light, and the parameter may significantly change an output value of the differential filter, when the image data acquired by capturing the image with the near-infrared light have a difference greater than that between a value indicating a vein portion and a value indicating a non-vein portion.

## EFFECT OF THE INVENTION

According to embodiments of the present invention, presence of a pseudo-vein pattern intentionally produced on a body surface can be determined.

## BRIEF DESCRIPTION OF THE DRAWINGS

FIG. 1 is an explanatory diagram illustrating captured images of a finger surface using visible light and near-infrared light;

FIG. 2 is an explanatory diagram illustrating a relation between captured images of the finger surface using the visible light as well as the near-infrared light and a Log filter output;

FIG. 3 is an explanatory diagram illustrating a vein pattern management system according to one embodiment of the present invention;

FIG. 4 is a block diagram illustrating a hardware configuration of a vein pattern registration apparatus according to the embodiment;

FIG. 5 is a block diagram illustrating a configuration of the vein pattern registration apparatus according to the embodiment;

FIG. 6 is a block diagram illustrating a configuration of a vein pattern authentication apparatus according to the embodiment;

FIG. 7 is a flowchart illustrating a skeleton extracting method according to the embodiment; and

FIG. 8 is an explanatory diagram illustrating a pseudo-vein pattern drawn on a finger surface.

DESCRIPTION OF REFERENCE NUMERALS

| | |
|---|---|
| 10: | vein pattern management system |
| 12: | network |
| 14: | removable storage medium |
| 20: | vein pattern registration apparatus |
| 30: | vein pattern authentication apparatus |
| 201: | CPU |
| 203: | ROM |
| 205: | RAM |
| 207: | bus |
| 211: | imaging device |
| 213: | input device |
| 215: | output device |
| 217: | storage device |
| 219: | drive |
| 221: | communication device |
| 231, 301: | imaging unit |
| 233, | 303: radiation unit |
| 235, | 305: near-infrared light radiation unit |
| 237, | 307: visible light radiation unit |
| 239,309: | near-infrared light |
| 241, 311: | visible light |
| 243,313: | optical lens |
| 245,315: | imaging data generation unit |
| 251,321: | vein pattern extraction unit |
| 253, 323: | correlation coefficient calculation unit |
| 261,331: | pseudo-vein pattern determination unit |
| 271,341: | vein pattern registration unit |
| 273,343: | storage unit |
| 275: | registered vein pattern disclosure unit |
| H: body | surface |

BEST MODE FOR CARRYING OUT THE INVENTION

(FIRST EMBODIMENT)

Hereinafter, preferred embodiments of the present invention will be described in detail with reference to the appended drawings. Note that, in this specification and the appended drawings, structural elements that have substantially the same function and structure are denoted with the same reference numerals, and repeated explanation of these structural elements is omitted.

Although, in a later description, the present invention will be described in connection with an example of vein patterns of fingers, the present invention is not limited to this example.

<Pseudo-Vein Pattern>

A pseudo-vein pattern intentionally formed on a finger surface will be described as an example of pseudo-vein patterns in preparation for a description of a vein pattern management system according to a first embodiment of the present invention.

In biometric authentication with finger vein pattern, although impersonation is difficult because a vein pattern itself is located inside of a finger, it is also difficult, in extraction of the vein pattern, to determine whether an extracted vein pattern is located inside of the finger. Since a vein per se absorbs near-infrared light, the vein is imaged as a dark shadow while capturing an image of a body surface, and if a pseudo-vein pattern is drawn on the body surface with a component, which has absorbency similar to that of the vein, the pseudo-vein pattern might be indistinguishable from the vein pattern.

Since the near-infrared light is permeable to body tissue, on one hand, and is absorbable in hemoglobin in blood (reduced hemoglobin), on the other hand, veins distributed inside of a finger, a palm of a hand, or a back of a hand appear as shadows in an image when the near-infrared light is radiated to the finger, the palm of the hand, or the back of the hand. The shadows of the vein appearing on the image are referred to as a vein pattern.

FIG. 8 is an explanatory diagram illustrating a pseudo-vein pattern drawn on a finger surface. The upper part of FIG. 8 represents a case in which a pseudo-vein pattern is directly drawn on a finger surface with a permanent pen, and the lower part of FIG. 8 represents a case in which no pseudo-vein patterns are drawn on the finger surface. In addition, in either of the upper and lower parts, there are shown from left to right a captured image with visible light, a captured image with near-infrared light, and an image subject to a threshold process of an output of a Laplacian of Gaussian (Log) filter that is a kind of differential filters, respectively.

The threshold process as used herein refers to a process in which predetermined upper and lower threshold values are assigned to an output value of a Log filter and the output value is set to zero if the output value is less than the lower threshold value and the output value is set to the upper threshold value if the output value is greater than the upper threshold value.

Since an ink component of the permanent pen has a light absorption property similar to that of reduced hemoglobin in a vein, the pseudo-vein pattern drawn with the permanent pen is left in an intermediate image not yet subject to a thinning process as a vein pattern, as shown in top right and bottom right ends of FIG. 8, and is ultimately recognized as a vein in the finger.

In order to solve such problems, the inventors of this application has been dedicated to developing so that the inventor has contrived a vein pattern management system, an vein pattern registration apparatus, a vein pattern authentication apparatus, a vein pattern registration method, a vein pattern authentication method, a program, and a vein data configuration.

<The Embodiment>

(Image Capturing Using Visible Light and Near-infrared Light)

Referring to FIG. 1, a result of capturing images of a finger, which is an object to be imaged, with visible light and near-infrared light will now be described in detail. FIG. 1 is an explanatory diagram illustrating captured images of a finger surface with the visible light and the near-infrared light. From left to right in FIG. 1, there are shown a capturing result with the visible light in the absence of a pseudo-vein pattern on the finger surface, a capturing result with the near-infrared light in the absence of the pseudo-vein pattern on the finger surface, a capturing result with the visible light in the presence of the pseudo-vein pattern on the finger surface, and a capturing result with the near-infrared light in the presence of the pseudo-vein pattern on the finger surface.

With reference to capturing results in the absence of the pseudo-vein pattern, it can be seen that a crimp or the like on the finger surface in case of the capturing result for the visible light is not imaged on the capturing result for the near-infrared light, and that a finger vein portion is imaged as a dark shadow on the capturing result for the near-infrared light. On the contrary, it can be seen that when a pseudo-vein pattern has been intentionally drawn on the finger surface by a permanent pen, a pseudo-vein pattern are clearly imaged on the capturing results for both the visible light and the near-infrared light.

Next, referring to FIG. 2, a relation between captured images of a finger surface using visible light and near-infrared light and a differential filter output will be described. FIG. 2 is an explanatory diagram illustrating the relation between the captured images of the finger surface using the visible light as well as the near-infrared light and an output of a Log filter, which is a kind of differential filters.

The upper part of FIG. 2 shows, sequentially left to right, a capturing result with visible light in the absence of a pseudo-vein pattern on a finger surface, a capturing result with near-infrared light in the absence of the pseudo-vein pattern on the finger surface, a capturing result with the visible light in the presence of the pseudo-vein pattern on the finger surface, and a capturing result with the near-infrared light in the presence of the pseudo-vein pattern on the finger surface. In addition, the lower part of FIG. 2 shows, sequentially from left to right, a Log filter output for a captured image with the visible light in the absence of the pseudo-vein pattern on the finger surface, a Log filter output for a captured image with the near-infrared light in the absence of the pseudo-vein pattern on the finger surface, a Log filter output for a captured image with the visible light in the presence of the pseudo-vein pattern on the finger surface, and a Log filter output for a captured image with the near-infrared light in the presence of the pseudo-vein pattern on the finger surface. In the lower part of FIG. 2, an identical Log filter process has been applied to each of the captured images with visible light and the near-infrared light.

In the absence of the pseudo-vein pattern, a crimp or the like, which is located on the finger surface, has been output as a white shadow in the Log filter output for the captured image with the visible light, and a vein pattern of a finger vein has been output as a white shadow in the Log filter output for the captured image with the near-infrared light. On the contrary, a pseudo-vein pattern has been output as a white shadow in both of Log filter outputs for the captured image with the visible light and the captured image with the near-infrared light.

As can be clearly seen by comparing lower images with each other, there is no similarity between the Log filter output for the captured image with the visible light and the Log filter output for the captured image with the near-infrared light in the absence of the pseudo-vein pattern on the finger surface, and there is a significant similarity between the Log filter output for the captured image with the visible light and the Log filter output for the captured image with the near-infrared light in the presence of the pseudo-vein pattern on the finger surface.

Thus in the absence of the pseudo-vein pattern on the finger surface, the Log filter output for the captured image with the visible light and the Log filter output for the captured image with the near-infrared light have a low correlation, and in the presence of the pseudo-vein pattern on the finger surface, the Log filter output for the captured image with the visible light and the Log filter output for the captured image with the near-infrared light have a high correlation.

The inventor of the present invention has been dedicated to intensive study based on the above-mentioned knowledge so that the inventor has conceived that it is possible to determine presence of a pseudo-vein pattern intentionally formed on a finger surface by capturing an image of a finger surface with visible light as well as capturing an image of the finger surface with near-infrared light in related art and calculating a correlation between a differential filter output for the captured image with the near-infrared light and a differential filter output for the captured image with the visible light.

(Vein Pattern Management System)

Next, referring to FIG. 3, a vein pattern management system 10 according to this embodiment will be described in detail. FIG. 3 is an explanatory diagram illustrating the vein pattern management system 10 according to this embodiment.

As shown in FIG. 3, the vein pattern management system 10 include, for example, a vein pattern registration apparatus 20, and a plurality of vein pattern authentication apparatuses 30A, 30B, ..., which are connected to the vein pattern registration apparatus 20 via a network 12.

The network 12 is a communication line network that connects the vein pattern registration apparatus 20 and a vein pattern authentication apparatus 30 such that they can communicate in either unidirection or bidirection. The network 12 may include, for example, public network, such as Internet, telephone network, satellite communication network, or multicasting network, private network, such as Wide Area Network (WAN), Local Area Network (LAN), Internet Protocol-Virtual Private Network (IP-VPN), Ethernet (registered trademark), or wireless LAN, and the like, and is limited neither to wired network nor wireless network.

The vein pattern registration apparatus 20 is operable to radiate light of a predetermined wavelength to a body surface of an individual desiring to register his/her vein pattern, capture an image of the body surface, extract a vein pattern from the captured image data, and register the extracted vein pattern as personal identity information. The vein pattern registration apparatus 20 is also operable to determine presence of a pseudo-vein pattern intentionally formed on the body surface and determine whether the extracted vein pattern should be registered or not. In addition, the vein pattern registration apparatus 20 may disclose registered vein patterns, which have been registered as the personal identity information, as required by the vein pattern authentication apparatus 30 to be described later.

The vein pattern authentication apparatuses 30A and 30B are operable to radiate light of the predetermined wavelength to a body surface of an individual desiring to register his/her vein pattern, capture an image of the body surface, extract a vein pattern from the captured image data, and compare the extracted vein pattern with already registered vein patterns to authenticate the individual. The vein pattern authentication apparatus 30 is also operable to

determine presence of a pseudo-vein pattern intentionally formed on the body surface and determine whether the extracted vein pattern should be authenticated or not. In addition, the vein pattern authentication apparatuses 30A and 30B may request the vein pattern registration apparatus 20 to disclose the already registered vein patterns.

It is noted that the vein pattern registration apparatus 20 and the vein pattern authentication apparatus 30A and 30B may be connected via the network 12 as shown in the figures, or may be directly connected via a Universal Serial Bus (USB) port, an IEEE 1394 port, such as an i.LINK, a Small Computer System Interface (SCSI) port, a RS-232C port, or the like, not via the network 12.

Although, in FIG. 3, there is only one vein pattern registration apparatus 20 connected to a network 12, this embodiment is not intended to be limited to a configuration as described above, but may allow a plurality of vein pattern registration apparatuses 20 to be connected on the network 12. Similarly, in FIG. 3, there are only two vein pattern authentication apparatuses 30 which are connected to the network 12, and a plurality of vein pattern authentication apparatuses 30 may be connected on the network 12.

(Configuration of Vein Pattern Registration Apparatus 20)

Referring to FIG. 4, a hardware configuration of a vein pattern registration apparatus 20 according to this embodiment will be described in detail. FIG. 4 is a block diagram illustrating the hardware configuration of the vein pattern registration apparatus 20 according to this embodiment.

As shown in FIG. 4, the vein pattern registration apparatus 20 mainly includes Central Processing Unit (CPU) 201, Read Only Memory (ROM) 203, Random Access Memory (RAM) 205, a bus 207, an imaging device 211, an input device 213, an output device 215, a storage device 217, a drive 219, and a communication device 221.

CPU 201 serves as a computing device and a controller for controlling all or a part of operations in the vein pattern registration apparatus 20 in accordance with various programs recorded in ROM 203, RAM 205, the storage device 217 or a removable recording medium 14. ROM 203 stores programs, operational parameters, and the like used by CPU 201. RAM 205 temporarily stores a program for use in execution by CPU 201, parameters that change appropriately in the execution of the program, and the like. CPU, ROM, and RAM are connected with each other via the bus 207 formed by an internal bus, such as a CPU bus.

The imaging device 211 is a device that captures an image of a body surface to generate image data under control of CPU 201. The imaging device 211 includes, for example, a radiation device for radiating light of a predetermined wavelength and a focusing device, such as an optical lens, for focusing light transmitting through the body surface. The radiation device includes a light source emitting the light of the predetermined wavelength and radiates the light of the predetermined wavelength based on a control signal from CPU 201. The focusing device collects the light radiated from the radiation device and generates the image data.

The input device 213 includes, for example, an operation means, such as mouse, a keyboard, a touch panel, a button, a switch, and a lever, which is operated by a user, and an audio input means, such as a microphone and a headset. In addition, the input device 213 may be, for example, a remote control means (what is called remote controller) using infrared radiation or other radio waves, or may be an external connection device, such as a mobile telephone and PDA, adapted to the operation of the vein pattern registration apparatus 20. Furthermore, the input device 213 may include, for example, an input control circuit or the like, for generating an input signal based on information input by the user using the above-mentioned operation means and audio input means and outputting the input signal to CPU 201. The user of the vein pattern registration apparatus 20 can input various data and instruct a processing operation to the vein pattern registration apparatus 20 by operating the input device 213.

The output device 215 includes, for example, a display device, such as a Cathode Ray Tube (CRT) display device, a Liquid Crystal Display (LCD) device, a Plasma Display Panel (PDP) device, an Electro-Luminescence (EL) display device and a lamp, an audio output device, such as a speaker and head phones, a printer, a mobile phone, a facsimile machine, and the like, which are capable of visually or audibly communicating acquired information to the user.

The storage device 217 is a data storing device, which is configured as an example of a storage unit of the vein pattern registration apparatus 20 according to this embodiment, and includes, for example, a magnetic storage device, such as a hard disk drive (HDD), a semiconductor storage device, an optical storage device, a magnetic optical storage device, or the like. The storage device 217 stores a wide variety of data, such as programs executed by CPU 201, various data, and various types of data acquired from an outside.

The drive 219 is a reader/writer for a storing medium and may be embedded in or attached externally to the vein pattern registration apparatus 20. The drive 219 reads out information recorded in the removable recording medium 14, such as an attached magnetic disk, optical disk, magnetic optical disk, or semiconductor memory, and outputs the information to RAM 205. In addition, the drive 219 is capable of writing recordings to the removable recording medium 14, such as the attached magnetic disk, optical disk, magnetic optical disk, or semiconductor memory. The removable recording medium 14 includes, for example, a DVD medium, a HD-DVD medium, a Blu-ray medium, CompactFlash (CF) (registered trademark), a memory stick, a Secure Digital (SD) memory card, or the like. In addition, the removable

recording medium 14 may be, for example, in a form of an Integrated Circuit (IC) card equipped with a non-contact IC chip, an electronic device, or the like.

The communication device 221 is a communication interface, which include, for example, a communication device for connecting to a communication network 12. The communication device 221 is made in a form of a communication card for use in wired or wireless Local Area Network (LAN), Bluetooth, or Wireless USB (WUSB), a router for use in optical communication, a router for use in Asymmetric Digital Subscriber Line (ADSL), a modem for use in various communication environments, or the like. This communication device 221 is capable of sending/receiving signals and the like to/from other vein pattern registration devices 20 and other vein pattern authentication devices 30. In addition, the network 12 connected to the communication device 221 is formed by networks and the like connected via wired or wireless connection, and may be configured, for example, as Internet, home LAN, infrared communication, satellite communication, or the like.

With a configuration as described above, the vein pattern registration apparatus 20 can radiate light of a predetermined wavelength to a body surface of an individual desiring to register his/her vein pattern, capture an image of the body surface, extract a vein pattern from the captured image data, and register the extracted vein pattern as personal identity information. In addition, the vein pattern registration apparatus 20 can send/receive data to/from the vein pattern authentication apparatus 30 directly connected to the vein pattern registration apparatus 20 or the vein pattern authentication apparatus 30 connected to the network 12, and retrieve information stored in the vein pattern registration apparatus 20 using the removable recording medium 14.

An example of a possible hardware configuration for implementing functions of vein pattern registration apparatus 20 according to this embodiment has been described above. Each of the above components may be configured using a general purpose member, or may be configured with a dedicated hardware for a function of each component. Thus, the hardware configuration used herein can be appropriately modified depending on state of the art at the time of implementing this embodiment.

A description of a hardware configuration of the vein pattern authentication apparatus 30 is omitted, since the hardware configuration of the vein pattern authentication apparatus 30 is substantially identical to that of the vein pattern registration apparatus 20.

Next, referring to FIG. 5, a configuration of a vein pattern registration apparatus 20 according to this embodiment will be described in detail. FIG. 5 is a block diagram illustrating the configuration of the vein pattern registration apparatus 20 according to this embodiment.

As shown in FIG. 5, the vein pattern registration apparatus 20 according to this embodiment includes, for example, an imaging unit 231, a vein pattern extraction unit 251, a pseudo-vein pattern determination unit 261, a vein pattern registration unit 271, a storage unit 273, and a registered vein pattern disclosure unit 275.

The imaging unit 231 captures an image of a body surface H of an individual desiring to register his/her vein pattern and generates imaging data. The imaging unit 231 includes, for example, a radiation unit 233 radiating light of a predetermined wavelength, an optical lens 243 focusing light transmitting through the body surface H, and an imaging data generation unit 245 generating imaging data based on the focused light.

The radiation unit 233 includes a light source for radiating light of a predetermined wavelength to a body surface H and includes, for example, a near-infrared light radiation unit 235 and a visible light radiation unit 237. The near-infrared light radiation unit 235 includes, for example, a halogen lamp, a light emitting diode, or the like, and radiates near-infrared light 239 having a wavelength of about 600 nm to 1,300 nm. Also, the visible light radiation unit 237 includes, for example, a xenon lamp or the like, and radiates visible light 241 having a wavelength of about 400 nm to 800 nm.

The optical lens 243 focuses the near-infrared light 239 and the visible light 241 transmitting through the body surface H, such as a finger surface, and forms an image on the imaging data generation unit 245. The optical lens 243 may be provided with two types of optical lenses including one for focusing the near-infrared light 239 and the other for focusing the visible light 241, or may be provided with a single optical lens capable of focusing both the near-infrared light 239 and the visible light 241.

The imaging data generation unit 245 generates near-infrared light imaging data and visible light imaging data based on transmitted light of the near-infrared light 239 and that of the visible light 241, respectively, which have been focused by the optical lens 243. The imaging data generation unit 245 includes, for example, a Charge Coupled Device (CCD) image sensor, a Complementary Metal Oxide Semiconductor (CMOS) image sensor, or the like and outputs the near-infrared light imaging data and the visible light imaging data to the vein pattern extraction unit 251 to be described later. In addition, the imaging data generation unit 245 may store the generated near-infrared light imaging data and visible light imaging data in the storage unit 273 to be described later. In storing in the storage unit 273, date of capture or time of capture may be associated to the generated near-infrared light imaging data and visible light imaging data. Furthermore, the generated near-infrared light imaging data and visible light imaging data may be in the form of a Red-Green-Blue (RGB) signal or may be image data of other colors, gray scale image data, or the like.

The vein pattern extraction unit 251 includes, for example, a function of performing a pre-process for vein pattern extraction on the near-infrared light imaging data transmitted from the imaging data generation unit 239, a function

of extracting a vein pattern, and a function of performing a post-process for the vein pattern extraction.

The pre-process for the vein pattern extraction includes, for example, a process for detecting a contour of a finger from near-infrared light imaging data and visible light imaging data and discriminating where the finger is located in the near-infrared light imaging data and the visible light imaging data, a process for rotating the near-infrared light imaging data or the visible light imaging data using the detected contour of the finger and correcting angles of the near-infrared light imaging data and the visible light imaging data (angles of captured image), and the like.

In addition, the vein pattern extraction may be achieved by applying a differential filter to the near-infrared light imaging data and the visible light imaging data, which have been subject to detecting the contour or correcting the angles. The differential filter is a filter that outputs a high value as an output value for an image of interest and its surrounding pixels at a portion where differences between the pixel of interest and its surrounding pixels, respectively, are large. In other words, the differential filter as used herein refers to a filter that enhances a line or an edge in an image by an operation using differences in gray level values between a pixel of interest and its surroundings.

In general, performing a filtering process on image data u(x, y) with a variable, which is a lattice point (x, y) on a two-dimensional plane, using a filter h(x, y) results in image data v(x, y), as shown in the following Equation. In the following Equation 1, * denotes convolution.

$$\begin{aligned} v(x,y) &= u(x,y) * h(x,y) \\ &= \sum_{m_1}\sum_{m_2} h(m_1,m_2)u(x-m_1,y-m_2) \\ &= \sum_{m_1}\sum_{m_2} u(m_1,m_2)h(x-m_1,y-m_2) \end{aligned} \tag{1}$$

In the vein pattern extraction according to this embodiment, a derivative filter, such as a first order spatial derivative filter or a second order spatial derivative filter may be used as the above-mentioned differential filter. The first order spatial derivative filter refers to a filter that, for a pixel of interest, calculates a difference in gray scale levels between the pixel of interest and its horizontally adjacent pixel or its vertically adjacent pixel, and the second order spatial derivative filter refers to a filter that extracts a portion having an increased variation in differences in gray scale values for a pixel of interest.

For example, the following Laplacian of Gaussian (Log) filter can be used as the above-mentioned second order spatial derivative filter. The Log filter (Equation 3) can be written as a second order derivative of a Gaussian filter (Equation 2), which is a smoothing filter using a Gauss function. In the following Equation 2, σ represents a standard deviation of the Gauss function, in other words, a variable representing a degree of smoothing for the Gaussian filter. Furthermore, σ in the following Equation 3 is also a parameter, which represent a standard deviation of the Gauss function, as is the case with Equation 2, and changing a value of σ can cause an output property (output value) to change in case of performing a Log filtering process.

$$h_{gauss}(x,y) = \frac{1}{2\pi\sigma^2}\exp\left\{-\frac{(x^2+y^2)}{2\sigma^2}\right\} \tag{2}$$

$$h_{Log}(x,y) = \nabla^2 \cdot h_{gauss}(x,y)$$
$$= (\frac{\partial^2}{\partial x^2} + \frac{\partial^2}{\partial y^2}) h_{gauss}$$
$$= \frac{(x^2 + y^2 - 2\sigma^2)}{2\pi\sigma^6} \exp\left\{ -\frac{(x^2 + y^2)}{2\sigma^2} \right\} \quad (3)$$

Also the above-described post-process for the vein pattern extraction may include, for example, a threshold process performed on image data, which has been subject to a differential filter, a binarization process, a thinning process, and the like. After having passed through the post-process, a skeleton of the vein pattern can be extracted.

The vein pattern extraction unit 251 transmits the vein pattern or the skeleton thus extracted to a correlation coefficient calculation unit 253 to be described later. The vein pattern extraction unit 251 may also store the extracted vein pattern or skeleton in the storage unit 273 to be described later. The vein pattern extraction unit 251 may store a parameter, intermediate results during the processes, and the like, which have been generated to perform each of the above-mentioned processes, in the storage unit 273.

In addition, the vein pattern extraction unit 251 further includes a correlation coefficient calculation unit 253 calculating a correlation coefficient representative of a similarity between a near-infrared light vein pattern and a visible light vein pattern. The correlation coefficient calculation unit 253 calculates the correlation coefficient between the near-infrared light vein pattern and the visible light vein pattern using the following Equation 4. The correlation coefficient is a statistical indicator, which indicates a similarity between two pieces of data: $x = \{x_i\}$ and $y = \{y_i\}$, and has a real number value from -1 to 1. When the correlation coefficient has a value close to 1, it indicates that the pieces of data are similar with each other, whereas when the correlation coefficient has a value close 0, it indicates that the two pieces of data are not similar with each other. In addition, when the correlation coefficient has a value close -1, it indicates a case where the two pieces of data have opposite signs to each other.

$$r = \frac{\sum_i (x_i - \bar{x})(y_i - \bar{y})}{\sqrt{\sum_i (x_i - \bar{x})^2} \sqrt{\sum_i (y_i - \bar{y})^2}} \quad (4)$$

x : Average of Data x

$\bar{y}$: Average of Data y

The correlation coefficient calculation unit 253 transmits a correlation coefficient between a near-infrared light vein pattern and a visible light vein pattern, which has been calculated, for example, based on Equation 4, to a pseudo-vein pattern determination unit 261 to be described later. The correlation coefficient calculation unit 253 may also store the calculated correlation coefficient in the storage unit 273.

The pseudo-vein pattern determination unit 261 determines presence of a pseudo-vein pattern intentionally formed on a part of a body surface H based on the correlation coefficient transmitted from the correlation coefficient calculation unit 253 in the vein pattern extraction unit 251. In particular, the pseudo-vein pattern determination unit 261 determines the presence of the pseudo-vein pattern by comparing the correlation coefficient transmitted from the correlation coefficient calculation unit 253 with a predetermined threshold value. The threshold value may be, for example, a value calculated from a prior determination test using multiple estimation data or may be a value specific to a particular individual.

Furthermore, as shown in FIG. 2, for example, when there are no pseudo-vein patterns, a correlation between the near-infrared light vein pattern and the visible light vein pattern is low. Thus a correlation coefficient is supposed to have a value close to zero. Whereas when there is a pseudo-vein pattern, a correlation between the near-infrared light vein pattern and the visible light vein pattern is high so that a correlation coefficient is supposed to have a value close to 1. As a result, it is possible to set the threshold value to 0.5, for example.

The pseudo-vein pattern determination unit 261 determines that a pseudo-vein pattern has been formed on a

part of the body surface H when the correlation coefficient transmitted from the correlation coefficient calculation unit 253 is higher than a predetermined threshold value and determines that a pseudo-vein pattern has not been formed on a part of the body surface H when the correlation coefficient is lower than the predetermined threshold value.

The pseudo-vein pattern determination unit 261 transmits a determination result to the vein pattern registration unit 271. The pseudo-vein pattern determination unit 261 may also store the determination result in the storage unit 273. Furthermore, in storing in the storage unit, the vein pattern that has been subject to the determination and the determination result may be stored in association with each other.

The vein pattern registration unit 271 registers a generated near-infrared light vein pattern as a template based on the determination result transmitted from the pseudo-vein pattern determination unit 261. In particular, when the determination result is transmitted from the pseudo-vein pattern determination unit 261, indicating that there is not presence of a pseudo-vein pattern, the vein pattern registration unit 271 stores the near-infrared light vein pattern transmitted from the vein pattern extraction unit 251 as a registered vein pattern in the storage unit 273. To the contrary, when the determination result is transmitted from the pseudo-vein pattern determination unit 261, indicating that there is presence of a pseudo-vein pattern, the vein pattern registration unit 271 does not register the extracted near-infrared light vein pattern and finishes a registration process. In registration of the registered vein pattern, not only the near-infrared light vein pattern is stored, but also other data for identifying an individual (for example, fingerprint data, face image data, iris data, voiceprint data, or the like) having the vein pattern may be stored in association with the near-infrared light vein pattern. Moreover, the registered vein pattern to be registered as the template may contain, for example, header information in conformity to a standard, such as a Common Biometric Exchange File Format (CBEFF) framework.

The storage unit 273 stores a registered vein pattern, which is requested to be registered from the vein pattern registration unit 271, or other data associated to the registered vein pattern. In addition to these data, imaging data generated by the imaging data generation unit 245, a vein pattern extracted by the vein pattern extraction unit 251, or the like may also be stored. Furthermore, in addition to these data, the vein pattern registration apparatus 20 can cause various parameters, intermediate results, and the like, which are needed to be stored in performing some processes, or a variety of databases and the like to be appropriately stored. This storing unit 273 can be freely read from/written to by the imaging unit 231, vein pattern extraction unit 251, pseudo-vein pattern determination unit 261, vein pattern registration unit 271, and the like.

The registered vein pattern disclosure unit 275 may disclose a registered vein pattern stored in the storage unit 273, for example, as required by the vein pattern authentication apparatus 30 connected to the vein pattern registration apparatus 20.

It is noted that the vein pattern registration apparatus 20 according to this embodiment may be implemented in various apparatuses, such as an information processing apparatus including a computer or a server, a mobile terminal or a personal digital assistant (PDA) including a mobile telephone or PHS, an automated teller machine (ATM), an entrance and exit control apparatus, and the like, for example.

Although in the above description, the registered vein pattern to be registered as the template has been described in a case of storing the pattern within the vein pattern registration apparatus 20, the registered vein pattern may be stored in a recording medium, such as DVD media, HD-DVD media, Blu-ray media, CompactFlash (registered trademark), memory stick, SD memory card, or the like, an IC card equipped with a non-contact IC chip, an electronic equipment, and the like.

An example of functions of vein pattern registration apparatus 20 according to this embodiment has been described above. Each of the above components may be configured using a general purpose member or circuit, or may be configured with a dedicated hardware for a function of each component. In addition, a function of each component may be achieved by only CPU or the like. Thus, a configuration used herein can be appropriately modified depending on state of the art at the time of implementing this embodiment.

(Structure of Vein Pattern Authentication Apparatus 30)

Next, referring to FIG. 6, a structure of a vein pattern authentication apparatus 30 according to this embodiment will be described in detail. FIG. 6 is a block diagram illustrating the structure of the vein pattern authentication apparatus 30 according to this embodiment.

As shown in FIG. 6, the vein pattern authentication apparatus 30 according to this embodiment includes, for example, an imaging unit 301, a vein pattern extraction unit 321, a pseudo-vein pattern determination unit 331, a vein pattern authentication unit 341, and a storage unit 343.

The imaging unit 301 captures an image of a body surface H of an individual desiring to authenticate his/her vein pattern and generates imaging data. The imaging unit 301 includes, for example, a radiation unit 303 radiating light of a predetermined wavelength, an optical lens 313 focusing light transmitting through the body surface H, and an imaging data generation unit 315 generating imaging data based on the focused light.

The radiation unit 303 includes a light source for radiating light of a predetermined wavelength to a body surface

H and includes, for example, a near-infrared light radiation unit 305 and a visible light radiation unit 307. The near-infrared light radiation unit 305 includes, for example, a halogen lamp, a light emitting diode, or the like, and radiates near-infrared light 309 having a wavelength of about 600 nm to 1,300 nm. Also, the visible light radiation unit 307 includes, for example, a xenon lamp or the like, and radiates visible light 311 having a wavelength of about 400 nm to 800 nm.

The optical lens 313 focuses the near-infrared light 309 and the visible light 311 transmitting through the body surface H, such as a finger surface, and forms an image on the imaging data generation unit 315. The optical lens 313 may be provided with two types of optical lenses including one for focusing the near-infrared light 309 and the other for focusing the visible light 311, or may be provided with a single optical lens capable of focusing both of the near-infrared light 309 and the visible light 311.

The imaging data generation unit 315 generates near-infrared light imaging data and visible light imaging data based on transmitted light of the near-infrared light 309 and that of the visible light 311, respectively, which have been focused by the optical lens 313. The imaging data generation unit 315 includes, for example, a CCD image sensor, a CMOS image sensor, or the like and outputs the near-infrared light imaging data and the visible light imaging data to the vein pattern extraction unit 321 to be described later. In addition, the imaging data generation unit 315 may store the generated near-infrared light imaging data and visible light imaging data in the storage unit 343 to be described later. In storing in the storage unit 343, date of capture or time of capture may be associated to the generated near-infrared light imaging data and visible light imaging data. Furthermore, the generated near-infrared light imaging data and visible light imaging data may be in the form of a RGB signal or may be image data of other colors, gray scale image data, or the like.

The vein pattern extraction unit 321 includes, for example, a function of performing a pre-process for vein pattern extraction on the near-infrared light imaging data and the visible light imaging data transmitted from the imaging data generation unit 315, a function of extracting a vein pattern, and a function of performing a post-process for the vein pattern extraction.

In this case, the pre-process for the vein pattern extraction includes, for example, a process for detecting a contour of a finger from near-infrared light imaging data and visible light imaging data and discriminating where the finger is located in the near-infrared light imaging data and the visible light imaging data, a process for rotating the near-infrared light imaging data or the visible light imaging data using the detected contour of the finger and correcting angles of the near-infrared light imaging data and the visible light imaging data (angles of captured image), and the like.

In addition, the vein pattern extraction may be achieved by applying a differential filter to the near-infrared light imaging data and the visible light imaging data, which have been subject to detecting the contour or correcting the angles. The differential filter is a filter that outputs a high value as an output value for a pixel of interest and its surrounding pixels at a portion where differences between the pixel of interest and its surrounding pixels, respectively, are large. In other words, the differential filter as used herein refers to a filter that enhances a line or an edge in an image by an operation using differences in grey level values between a pixel of interest and its surroundings.

In general, performing a filtering process on image data u(x, y) with a variable, which is a lattice point (x, y) on a two-dimensional plane, using a filter h(x, y) results in image data v(x, y), as shown in the following Equation 5. In the following Equation 5,

* denotes convolution.

$$\begin{aligned} v(x,y) &= u(x,y) * h(x,y) \\ &= \sum_{m_1}\sum_{m_2} h(m_1,m_2)u(x-m_1,y-m_2) \\ &= \sum_{m_1}\sum_{m_2} u(m_1,m_2)h(x-m_1,y-m_2) \end{aligned} \tag{5}$$

In the vein pattern extraction according to this embodiment, a derivative filter, such as a first order spatial derivative filter or a second order spatial derivative filter may be used as the above-mentioned differential filter. The first order spatial derivative filter refers to a filter that, for a pixel of interest, calculates a difference in gray scale levels between the pixel of interest and its horizontally adjacent pixel or its vertically adjacent pixel, and the second order spatial derivative filter refers to a filter that extracts a portion having an increased variation in differences in gray scale values for a pixel of interest.

For example, the following Laplacian of Gaussian (Log) filter can be used as the above-mentioned second

order spatial derivative filter. The Log filter (Equation 7) can be written as a second order derivative of a Gaussian filter (Equation 6), which is a smoothing filter using a Gauss function. In the following Equation 6, σ represents a standard deviation of the Gauss function, and in other words, a variable representing a degree of smoothing for the Gaussian filter. Furthermore, σ in the following Equation 7 is also a parameter, which represents a standard deviation of the Gauss function, as is the case with Equation 6, and changing a value of σ can cause an output property (output value) to change in case of performing a Log filtering process.

$$h_{gauss}(x,y) = \frac{1}{2\pi\sigma^2}\exp\left\{-\frac{(x^2+y^2)}{2\sigma^2}\right\} \quad (6)$$

$$\begin{aligned} h_{Log}(x,y) &= \nabla^2 \cdot h_{gauss}(x,y) \\ &= \left(\frac{\partial^2}{\partial x^2}+\frac{\partial^2}{\partial y^2}\right)h_{gauss} \\ &= \frac{(x^2+y^2-2\sigma^2)}{2\pi\sigma^6}\exp\left\{-\frac{(x^2+y^2)}{2\sigma^2}\right\} \end{aligned} \quad (7)$$

Also, the above-described post-process for the vein pattern extraction may include, for example, a threshold process performed on image data, which has been subject to a differential filter, a binarization process, a thinning process, and the like. After having passed through the post-process, a skeleton of the vein pattern can be extracted.

The vein pattern extraction unit 321 transmits the vein pattern or the skeleton thus extracted to a correlation coefficient calculation unit 323 to be described later. The vein pattern extraction unit 321 may also store the extracted vein pattern or skeleton in the storage unit 343 to be described later. The vein pattern extraction unit 321 may store a parameter, intermediate results during the processes, and the like, which have been generated to perform each of the above-mentioned processes, in the storage unit 343.

In addition, the vein pattern extraction unit 321 further includes a correlation coefficient calculation unit 323 calculating a correlation coefficient representative of a similarity between a near-infrared light vein pattern and a visible light vein pattern. The correlation coefficient calculation unit 323 calculates the correlation coefficient between the near-infrared light vein pattern and the visible light vein pattern using the following Equation 8. The correlation coefficient is a statistical indicator, which indicates a similarity between two pieces of data: $x = \{x_i\}$ and $y = \{y_i\}$, and has a real number value from -1 to 1. When the correlation coefficient has a value close to 1, it indicates that the pieces of data are similar with each other, whereas when the correlation coefficient has a value close 0, it indicates that the two pieces of data are not similar with each other. In addition, when the correlation coefficient has a value close -1, it indicates a case where the two pieces of data have opposite signs to each other.

$$r = \frac{\sum_i (x_i-\bar{x})(y_i-\bar{y})}{\sqrt{\sum_i (x_i-\bar{x})^2}\sqrt{\sum_i (y_i-\bar{y})^2}} \quad (8)$$

$\bar{x}$: Average of Data x

$\bar{y}$: Average of Data y

The correlation coefficient calculation unit 323 transmits a correlation coefficient between a near-infrared light vein pattern and a visible light vein pattern, which has been calculated, for example, based on Equation 8, to a pseudo-vein pattern determination unit 331 to be described later. The correlation coefficient calculation unit 323 may also store

the calculated correlation coefficient in the storage unit 343.

The pseudo-vein pattern determination unit 331 determines presence of a pseudo-vein pattern intentionally formed on a part of a body surface H based on the correlation coefficient transmitted from the correlation coefficient calculation unit 323 in the vein pattern extraction unit 321. In particular, the pseudo-vein pattern determination unit 331 determines the presence of the pseudo-vein pattern by comparing the correlation coefficient transmitted from the correlation coefficient calculation unit 323 with a predetermined threshold value. The threshold value may be, for example, a value calculated from a prior determination test using multiple estimation data or may be a value specific to a particular individual.

Furthermore, as shown in FIG. 2, for example, when there are no pseudo-vein patterns, a correlation between the near-infrared light vein pattern and the visible light vein pattern is low. Thus a correlation coefficient is supposed to have a value close to zero. Whereas when there is a pseudo-vein pattern, a correlation between the near-infrared light vein pattern and the visible light vein pattern is high so that a correlation coefficient is supposed to have a value close to 1. As a result, it is possible to set the threshold value to 0.5, for example.

The pseudo-vein pattern determination unit 331 determines that a pseudo-vein pattern has been formed on a part of the body surface H when the correlation coefficient transmitted from the correlation coefficient calculation unit 323 is higher than a predetermined threshold value and determines that a pseudo-vein pattern has not been formed on a part of the body surface H when the correlation coefficient is lower than the predetermined threshold value.

The pseudo-vein pattern determination unit 331 transmits a determination result to the vein pattern authentication unit 341. The pseudo-vein pattern determination unit 331 may also store the determination result in the storage unit 333. Furthermore, in storing in the storage unit, the vein pattern that has been subject to the determination and the determination result may be stored in association with each other.

The vein pattern authentication unit 341 performs authentication of a resulting near-infrared light vein pattern based on the determination result transmitted from the pseudo-vein pattern determination unit 331. In particular, when the determination result indicating "there is no pseudo-vein pattern present" is transmitted from the pseudo-vein pattern determination unit 331, for example, the vein pattern authentication unit 341 requests the vein pattern registration apparatus 20, for example, to disclose a registered vein pattern and compares the registered vein pattern acquired from the vein pattern registration apparatus 20 with the near-infrared light vein pattern transmitted from the vein pattern extraction unit 251. Such a comparison of the registered vein pattern with the near-infrared light vein pattern can be achieved, for example, by calculating the above-mentioned correlation coefficient and performing the comparison based on the calculated correlation coefficient. The vein pattern authentication unit 341 authenticates the near-infrared light vein pattern when a comparison result indicates that the registered vein pattern and the near-infrared light vein pattern are similar with each other and does not authenticate the near-infrared light vein pattern when they are not similar with each other.

To the contrary, when the determination result is transmitted from the pseudo-vein pattern determination unit 331, indicating that there is presence of a pseudo-vein pattern, the vein pattern authentication unit 341 does not perform and finishes an authentication process of the extracted near-infrared light vein pattern.

The storage unit 343 is capable of storing imaging data generated by the imaging data generation unit 315, the vein pattern extracted by the vein pattern extraction unit 321, or the like. Furthermore, in addition to these data, the vein pattern authentication apparatus 30 can cause various parameters, intermediate results, and the like, which are needed to be stored in performing some processes, or a variety of databases and the like to be appropriately stored. This storing unit 343 can be freely read from/written to by the imaging unit 301, vein pattern extraction unit 321, pseudo-vein pattern determination unit 331, vein pattern authentication unit 341, and the like.

The vein pattern authentication apparatus 30 according to this embodiment may be implemented in various apparatuses, such as an information processing apparatus including a computer or a server, a mobile terminal or a personal digital assistant (PDA) including a mobile telephone or PHS, an automated teller machine (ATM), an entrance and exit control apparatus, and the like, for example.

Although in the above description, the registered vein pattern is supposed to be acquired from the vein pattern registration apparatus 20, the authentication may be performed based on the registered vein pattern, which has been stored in a recording medium, such as DVD media, HD-DVD media, Blu-ray media, CompactFlash (registered trademark), memory stick, SD memory card, or the like, an IC card equipped with a non-contact IC chip, an electronic equipment, and the like. Furthermore, the registered vein pattern may be stored in the vein pattern authentication apparatus 30.

An example of functions of vein pattern authentication apparatus 30 according to this embodiment has been described above. Each of above components may be configured using a general purpose member or circuit, or may be configured with a dedicated hardware for a function of each component. In addition, a function of each component may be achieved by only CPU or the like. Thus, a configuration used herein can be appropriately modified depending on state of the art at the time of implementing this embodiment.

(Registration Method of Vein Pattern)

Next, referring to FIG. 7, a method for registering a vein pattern according to this embodiment will be described in detail. FIG. 7 is a flowchart illustrating a method for extracting a skeleton according to this embodiment.

In general, an image of a finger vein located in a finger is captured with near-infrared light only. The method for registering a vein pattern according to this embodiment, however, is **characterized in that** a process for extracting a finger vein pattern is performed by not only capturing an image of a finger vein with near-infrared light, but also capturing an image of a finger with visible light.

Firstly, an imaging unit 231 captures an image of a part of a body surface (for example, a finger surface) and an imaging data generation unit 245 in the imaging unit 231 generates near-infrared light imaging data (step S101). The imaging data generation unit 245 stores the generated near-infrared light imaging data in a storage unit 273, for example, in association with date of capture or time of capture, and transmits the generated near-infrared light imaging data to a vein pattern extraction unit 251.

The vein pattern extraction unit 251, to which the near-infrared light imaging data transmitted, performs a pre-process for skeleton extraction of a vein pattern on the near-infrared light imaging data, in which the pre-process includes a process for detecting a contour of a finger and discriminating a position of the finger, or a process for rotating the near-infrared light imaging data and correcting an angle of the near-infrared light imaging data (step S103).

Once the pre-process for the skeleton extraction has finished, the vein pattern extraction unit 251 then calculates a Log filter output by applying a Log filter process, which is a kind of differential filters, to the near-infrared light imaging data, which has been subject to the pre-process, to generate a near-infrared light vein pattern (step S105). After calculating an output value of the Log filter, the vein pattern extraction unit 251 stores the calculated output value of the Log filter (near-infrared light vein pattern) in the storage unit 273.

The imaging unit 231 then captures an image of the same part of the finger surface with visible light as that of the finger surface captured with the near-infrared light and the imaging data generation unit 245 in the imaging unit 231 generates visible light imaging data (step S107). The imaging data generation unit 245 stores the generated visible light imaging data in a storage unit 273, for example, in association with date of capture or time of capture, and transmits the generated visible light imaging data to the vein pattern extraction unit 251.

The vein pattern extraction unit 251, to which the visible light imaging data has been transmitted, performs a pre-process for the skeleton extraction of a vein pattern (step S109), including a process for detecting a contour of a finger from the visible light imaging data and discriminating where the finger is located in the visible light imaging data, a process for rotating the near-infrared light imaging data and correcting an angle of the near-infrared light imaging data, and the like.

Once the pre-process for the skeleton extraction has finished, the vein pattern extraction unit 251 then applies a Log filter process, which is a kind of differential filters, to the visible light imaging data, which has been subject to the pre-process, and calculates a Log filter output to generate a visible light vein pattern (step S111). The Log filter used for the visible imaging data is identical to the Log filter used for the near-infrared light imaging data. After calculating an output value of the Log filter, the vein pattern extraction unit 251 stores the calculated output value of the Log filter (visible light vein pattern) in the storage unit 273.

Once the calculation of the output value of the Log filter has finished for the near-infrared light imaging data and the visible light imaging data and the near-infrared light vein pattern and the visible light vein pattern, respectively, have been generated, a correlation coefficient calculation unit 253 in the vein pattern extraction unit 251 calculates a correlation coefficient between the near-infrared light vein pattern and the visible light vein pattern using, for example, the above-mentioned Equation 4 (step S113). Once the calculation of the correlation coefficient has finished, the correlation coefficient calculation unit 253 stores the calculated correlation coefficient in the storage unit 273 and transmits it to a pseudo-vein pattern determination unit 261.

The pseudo-vein pattern determination unit 261 determines presence of a pseudo-vein pattern on a part of a body surface (for example, a finger surface) based on the correlation coefficient transmitted from the correlation coefficient calculation unit 253. The determination is performed by determining whether the calculated correlation coefficient is less than a predetermined threshold value or equal to or greater than the predetermined threshold value (step S 115).

On one hand, the pseudo-vein pattern determination unit 261 determines that the pseudo-vein pattern is not present on the finger surface, which is an object to be imaged, and informs the vein pattern extraction unit 251 and the vein pattern registration unit 271 of this determination result. On receipt of the information of the determination result, the vein pattern extraction unit 251 applies a post-process, such as a threshold process, a binarization process, and a thinning process, to the near-infrared light vein pattern (step S117), and stores the near-infrared light vein pattern that has been subject to the post-process in the storage unit 273 as well as transmits the near-infrared light vein pattern to the vein pattern registration unit 271.

On the other hand, the pseudo-vein pattern determination unit 261 determines that the pseudo-vein pattern is present on the finger surface, which is an object to be imaged, and informs the vein pattern registration unit 271 of this

determination result.

When the vein pattern registration unit 271 is informed of a signal indicating that there are no pseudo-vein patterns present from the pseudo-vein pattern determination unit 261, the vein pattern registration unit 271 stores the near-infrared light vein pattern subject to the post-process and transmitted from the vein pattern extraction unit 251 as a registered vein pattern in a database (not shown) contained in the storage unit 273. In addition, the registered vein pattern may be associated with ID or other biometrics data of an individual, or the like.

Furthermore, when the vein pattern registration unit 271 is informed of a signal indicating that there is a pseudo-vein pattern present from the pseudo-vein pattern determination unit 261, the vein pattern registration unit 261 does not perform a registration process of the vein pattern and finishes a series of processes.

As described above, in the method for registering a vein pattern according to this embodiment, it is possible to determine presence of a pseudo-vein pattern intentionally formed on a part of a body surface by generating both imaging data with visible light and imaging data with near-infrared light and focusing attention on a correlation between a visible light vein pattern and a near-infrared light vein pattern. Since the method for registering the vein pattern according to this embodiment can determine presence of the pseudo-vein pattern before registering the vein pattern, possibility of storing unnecessary data in a database and the like, in which registered vein patterns are contained, is avoided, and it becomes easy to manage the registered vain patterns.

(Authentication Method of Vein Pattern)

Next, again referring to FIG. 7, a method for authenticating a vein pattern according to this embodiment will be described in detail.

In general, an image of a finger vein located in a finger is captured with near-infrared light only. A method for authenticating a vein pattern according to this embodiment is also **characterized in that** a process for extracting a finger vein pattern is performed by not only capturing an image of a finger vein with near-infrared light, but also capturing an image of a finger with visible light.

Firstly, an imaging unit 301 captures an image of a part of a body surface (for example, a finger surface) and an imaging data generation unit 309 in the imaging unit 301 generates near-infrared light imaging data (step S101). The imaging data generation unit 315 stores the generated near-infrared light imaging data in a storage unit 343, for example, in association with date of capture or time of capture, and transmits the generated near-infrared light imaging data to a vein pattern extraction unit 321.

The vein pattern extraction unit 321, to which the near-infrared light imaging data transmitted, performs a pre-process for skeleton extraction of a vein pattern on the near-infrared light imaging data, in which the pre-process includes a process for detecting a contour of a finger and discriminating a position of the finger, or a process for rotating the near-infrared light imaging data and correcting an angle of the near-infrared light imaging data (step S103).

Once the pre-process for the skeleton extraction has finished, the vein pattern extraction unit 321 then calculates a Log filter output by applying a Log filter process, which is a kind of differential filters, to the near-infrared light imaging data, which has been subject to the pre-process, to generate a near-infrared light vein pattern (step S105). After calculating an output value of the Log filter, the vein pattern extraction unit 321 stores the calculated output value of the Log filter (near-infrared light vein pattern) in the storage unit 343.

The imaging unit 301 then captures an image of the same part of the finger surface with visible light as that of the finger surface captured with the near-infrared light and the imaging data generation unit 315 in the imaging unit 301 generates visible light imaging data (step S107). The imaging data generation unit 315 stores the generated visible light imaging data in a storage unit 343, for example, in association with date of capture or time of capture, and transmits the generated visible light imaging data to the vein pattern extraction unit 321.

The vein pattern extraction unit 321, to which the visible light imaging data has been transmitted, performs a pre-process for the skeleton extraction of a vein pattern (step S109), including a process for detecting a contour of a finger from the visible light imaging data and discriminating where the finger is located in the visible light imaging data, a process for rotating the near-infrared light imaging data and correcting an angle of the near-infrared light imaging data, and the like.

Once the pre-process for the skeleton extraction has finished, the vein pattern extraction unit 321 then applies a Log filter process, which is a kind of differential filters, to the visible light imaging data, which has been subject to the pre-process, and calculates a Log filter output to generate a visible light vein pattern (step S111). The Log filter used for the visible imaging data is identical to the Log filter used for the near-infrared light imaging data. After calculating an output value of the Log filter, the vein pattern extraction unit 321 stores the calculated output value of the Log filter (visible light vein pattern) in the storage unit 343.

Once the calculation of the output value of the Log filter has finished for the near-infrared light imaging data and the visible light imaging data and the near-infrared light vein pattern and the visible light vein pattern, respectively, have been generated, a correlation coefficient calculation unit 323 in the vein pattern extraction unit 321 calculates a

correlation coefficient between the near-infrared light vein pattern and the visible light vein pattern using, for example, the above-mentioned Equation 8 (step S113). Once the calculation of the correlation coefficient has finished, the correlation coefficient calculation unit 323 stores the calculated correlation coefficient in the storage unit 343 and transmits it to a pseudo-vein pattern determination unit 331.

The pseudo-vein pattern determination unit 331 determines presence of a pseudo-vein pattern on a part of a body surface (for example, a finger surface) based on the correlation coefficient transmitted from the correlation coefficient calculation unit 323. This determination is performed by determining whether the calculated correlation coefficient is less than a predetermined threshold value or equal to or greater than the predetermined threshold value (step S 115).

On one hand, if the calculated correlation coefficient is less than a predetermined threshold value, the pseudo-vein pattern determination unit 331 determines that the pseudo-vein pattern is not present on the finger surface, which is an object to be imaged, and informs the vein pattern extraction unit 321 and the vein pattern authentication unit 341 of this determination result. On receipt of the information of the determination result, the vein pattern extraction unit 321 applies a post-process, such as a threshold process, a binarization process, and a thinning process, to the near-infrared light vein pattern (step S117), and stores the near-infrared light vein pattern that has been subject to the post-process in the storage unit 343 as well as transmits the near-infrared light vein pattern to the vein pattern authentication unit 341.

On the other hand, if the calculated correlation coefficient is equal to or greater than the predetermined threshold value, the pseudo-vein pattern determination unit 331 determines that the pseudo-vein pattern is present on the finger surface, which is an object to be imaged, and informs the vein pattern authentication unit 341 of this determination result.

When the vein pattern authentication unit 341 is informed of a signal indicating that there are no pseudo-vein patterns present from the pseudo-vein pattern determination unit 331, the vein pattern authentication unit 341 requests the vein pattern registration apparatus 20 to disclose a registered vein pattern. Once the registered vein pattern has been disclosed by a registered vein pattern disclosure unit 275 in the vein pattern registration apparatus 20, the vein pattern authentication unit 341 acquires and compares the disclosed registered vein pattern with the near-infrared light vein pattern, which has been subject to the post-process, transmitted from the vein pattern extraction unit 321. Comparison of the registered vein pattern with the near-infrared light vein pattern is performed, for example, using a method capable of quantitatively calculating similarity, such as above-mentioned correlation coefficient, between image data. The vein pattern authentication unit 341 authenticates the generated near-infrared light vein pattern when the registered vein pattern and the near-infrared light vein pattern are similar with each other, but the vein pattern authentication unit 341 does not authenticate the near-infrared light vein pattern when they are not similar with each other.

Furthermore, when the vein pattern authentication unit 341 is informed of a signal indicating that there is a pseudo-vein pattern present from the pseudo-vein pattern determination unit 331, the vein pattern authentication unit 331 does not perform an authentication process of the vein pattern and finishes a series of processes.

As described above, in the method for authenticating a vein pattern according to this embodiment, it is possible to determine presence of a pseudo-vein pattern intentionally formed on a part of a body surface by generating both imaging data with visible light and imaging data with near-infrared light and focusing attention on a correlation between a visible light vein pattern and a near-infrared light vein pattern. Since the method for authenticating the vein pattern according to this embodiment can determine presence of a pseudo-vein pattern before authenticating the vein pattern, it can previously prevent malicious users from impersonating others by repeating try and error to optimize a pseudo-vein pattern.

Although in the above-mentioned descriptions of the method for registering the vein pattern and the method for authenticating the vein pattern, it is described that first an image is captured with near-infrared light and then an image is captured with visible light, the image may be first captured with the visible light and then the image may be captured with the near-infrared light or the images may be simultaneously captured with the near-infrared light and the visible light. In addition, a pre-process for the skeleton extraction or a process for calculating an output of a Log filter may be simultaneously performed on both near-infrared light imaging data and visible light imaging data.

Although in the above-mentioned description, it is described that a correlation coefficient is calculated in order to achieve a correlation between a near-infrared light vein pattern and a visible light vein pattern, the correlation can be achieved, without being limited to this method, by any method that is capable of determining a similarity between two pieces of image data.

(Vein Data configuration)

Furthermore, according to an embodiment of the present invention, there is provided a vein data configuration including a vein data storage area containing data, which correspond to a vein pattern of an individual and are to be verified with image data acquired by capturing an image of a body surface of a portion of a living body with near-infrared light, and a correlation coefficient storage area containing a correlation coefficient between the image data acquired by capturing the image with the near-infrared light and image data acquired by capturing an image of the body surface with visible light.

The vein data storage area is an area containing, for example, a vein pattern that has been registered as a registered vein pattern by the vein pattern registration apparatus 20. The data contained in this vein data storage area are used, for example, by the vein pattern authentication apparatus 30 in authenticating a near-infrared light vein pattern captured.

The correlation coefficient storage area is an area in which a correlation coefficient is contained, which represents a similarity between a near-infrared light vein pattern acquired by capturing an image of the body surface of the individual with the near-infrared light and a visible light vein pattern acquired by capturing an image of the same part of the same individual with the visible light as in the case of the near-infrared light. The correlation coefficient contained in the correlation coefficient storage area is used, for example, by the vein pattern registration apparatus 20 or the vein pattern authentication apparatus 30 to determine presence of a pseudo-vein pattern formed on the body surface.

The above-mentioned vein data configuration may further include a parameter storage area containing a parameter, which changes an output property of a differential filter outputting a high output for an pixel that differs largely from its surrounding pixels, for each pixel constituting the image data acquired by capturing the image with the near-infrared light.

The parameter contained in the parameter storage area is a parameter for a differential filter used, for example, by the vein pattern registration apparatus 20 or the vein pattern authentication apparatus 30 in extracting a vein pattern from imaging data captured with near-infrared light or visible light, and the parameter significantly changes an output value of the differential filter, for example, when the image data acquired by capturing the image with the near-infrared light have a difference greater than that between a value indicating a vein portion and a value indicating a non-vein portion.

The above-mentioned parameter is separately contained for each type of differential filters and makes a pseudo-vein pattern formed on the body surface have a value such that the pseudo-vein pattern can be detected by the differential filter. For example, when a Log filter is used as the differential filter, a value, by which the Log filter can detect the pseudo-vein pattern, is contained in the parameter storage area. In this case, the value of the parameter to be contained is equal to or greater than 2.0.

The above-mentioned vein data configuration can be applied to, for example, a non-contact IC chip, or an IC card, such as a Subscriber Identity Module (SIM) card, used in a mobile telephone and the like. In addition, this vein data configuration can be applied to a recording medium, such as a DVD medium, a HD-DVD medium, a Blu-ray medium, CompactFlash (registered trademark), a memory stick, or a SD memory card.

It should be understood by those skilled in the art that various modifications, combinations, sub-combinations and alterations may occur depending on design requirements and other factors insofar as they are within the scope of the appended claims or the equivalents thereof.

For example, although in the above-mentioned embodiments, it has been described that a vein pattern registration apparatus 20 and a vein pattern authentication apparatus 30 are separately provided, respectively, a vein pattern management apparatus including functions of both a vein pattern registration apparatus 20 and a vein pattern authentication apparatus 30 may be provided.

Furthermore, although in the above-mentioned embodiments, it has been described that a transmissive imaging unit is provided each of a vein pattern registration apparatus 20 and a vein pattern authentication apparatus 30, a reflective imaging unit may be provided depending on a portion of a body surface to be captured.

## Claims

**1.** A vein pattern management system for registering and authenticating a vein pattern acquired by radiating light to a portion of a living body, comprising:

an imaging unit for capturing images of a body surface of the portion of the living body with near-infrared light and visible light, and generating near-infrared light imaging data and visible light imaging data, respectively;
a vein pattern extraction unit for extracting vein patterns from the near-infrared light imaging data and the visible light imaging data to generate a near-infrared light vein pattern and a visible light vein pattern, respectively;
a pseudo-vein pattern determination unit for determining presence of a pseudo-vein pattern intentionally formed on a part of the captured body surface by comparing the near-infrared light vein pattern with the visible light vein pattern;
a vein pattern registration unit for registering the near-infrared light vein pattern based on a determination result from the pseudo-vein pattern determination unit to generate a registered vein pattern; and
a vein pattern authentication unit for comparing a newly generated near-infrared light vein pattern with the registered vein pattern based on the determination result from the pseudo-vein pattern determination unit and authenticating the newly generated near-infrared vein pattern.

2. The vein pattern management system according to claim 1, wherein the pseudo-vein pattern determination unit determines the presence of the pseudo-vein pattern by calculating a correlation coefficient between the near-infrared light vein pattern and the visible light vein pattern.

3. The vein pattern management system according to claim 2, wherein the pseudo-vein pattern determination unit compares the calculated correlation coefficient with a predetermined threshold value for determination, determines that the pseudo-vein pattern is not present when the calculated correlation coefficient is less than the predetermined threshold value for determination, and determines that the pseudo-vein pattern is present when the calculated correlation coefficient is equal to or greater than the predetermined threshold value for determination.

4. The vein pattern management system according to claim 1, wherein the vein pattern extraction unit extracts the near-infrared light vein pattern and the visible light vein pattern using a differential filter that outputs a large value for a pixel having a large difference between the pixel and its surrounding pixels for a plurality of pixels constituting the near-infrared light imaging data and the visible light imaging data, respectively.

5. The vein pattern management system according to claim 4, wherein the differential filter is a derivative filter.

6. The vein pattern management system according to claim 5, wherein the differential filter is a Laplacian of Gaussian (Log) filter.

7. A vein pattern registration apparatus comprising:

an imaging unit for capturing images of a body surface of a portion of a living body with near-infrared light and visible light, and generating near-infrared light imaging data and visible light imaging data, respectively;
a vein pattern extraction unit for extracting vein patterns from the near-infrared light imaging data and the visible light imaging data to generate a near-infrared light vein pattern and a visible light vein pattern, respectively;
a pseudo-vein pattern determination unit for determining presence of a pseudo-vein pattern intentionally formed on a part of the captured body surface by comparing the near-infrared light vein pattern with the visible light vein pattern; and
a vein pattern registration unit for registering the near-infrared light vein pattern based on a determination result from the pseudo-vein pattern determination unit to generate a registered vein pattern.

8. The vein pattern registration apparatus according to claim 7, wherein the pseudo-vein pattern determination unit determines the presence of the pseudo-vein pattern by calculating a correlation coefficient between the near-infrared light vein pattern and the visible light vein pattern.

9. The vein pattern registration apparatus according to claim 8, wherein the pseudo-vein pattern determination unit compares the calculated correlation coefficient with a predetermined threshold value for determination, determines that the pseudo-vein pattern is not present when the calculated correlation coefficient is less than the predetermined threshold value for determination, and determines that the pseudo-vein pattern is present when the calculated correlation coefficient is equal to or greater than the predetermined threshold value for determination.

10. The vein pattern registration apparatus according to claim 7, wherein the vein pattern extraction unit extracts the near-infrared light vein pattern and the visible light vein pattern using a differential filter that outputs a large value for a pixel having a large difference between the pixel and its surrounding pixels for a plurality of pixels constituting the near-infrared light imaging data and the visible light imaging data, respectively.

11. The vein pattern registration apparatus according to claim 10, wherein the differential filter is a derivative filter.

12. The vein pattern registration apparatus according to claim 11, wherein the differential filter is a Laplacian of Gaussian (Log) filter.

**13.** A vein pattern authentication apparatus comprising:

an imaging unit for capturing images of a body surface of a portion of a living body with near-infrared light and visible light, and generating near-infrared light imaging data and visible light imaging data, respectively;
a vein pattern extraction unit for extracting vein patterns from the near-infrared light imaging data and the visible light imaging data to generate a near-infrared light vein pattern and a visible light vein pattern, respectively;
a pseudo-vein pattern determination unit for determining presence of a pseudo-vein pattern intentionally formed on a part of the captured body surface by comparing the near-infrared light vein pattern with the visible light vein pattern; and
a vein pattern authentication unit for comparing an already registered vein pattern with the near-infrared light vein pattern and authenticating the near-infrared light vein pattern based on a determination result from the pseudo-vein pattern determination unit.

**14.** The vein pattern authentication apparatus according to claim 13, wherein
the pseudo-vein pattern determination unit determines the presence of the pseudo-vein pattern by calculating a correlation coefficient between the near-infrared light vein pattern and the visible light vein pattern.

**15.** The vein pattern authentication apparatus according to claim 14, wherein
the pseudo-vein pattern determination unit compares the calculated correlation coefficient with a predetermined threshold value for determination, determines that the pseudo-vein pattern is not present when the calculated correlation coefficient is less than the predetermined threshold value for determination, and determines that the pseudo-vein pattern is present when the calculated correlation coefficient is equal to or greater than the predetermined threshold value for determination.

**16.** The vein pattern authentication apparatus according to claim 13, wherein
the vein pattern extraction unit extracts the near-infrared light vein pattern and the visible light vein pattern using a differential filter that outputs a large value for a pixel having a large difference between the pixel and its surrounding pixels for a plurality of pixels constituting the near-infrared light imaging data and the visible light imaging data, respectively.

**17.** The vein pattern authentication apparatus according to claim 16, wherein
the differential filter is a derivative filter.

**18.** The vein pattern authentication apparatus according to claim 17, wherein
the differential filter is a Laplacian of Gaussian (Log) filter.

**19.** The vein pattern authentication apparatus according to claim 13, wherein
the vein pattern authentication unit authenticates the near-infrared light vein pattern based on the registered vein pattern acquired from a vein pattern registration apparatus.

**20.** The vein pattern authentication apparatus according to claim 13, wherein
the vein pattern authentication unit authenticates the near-infrared light vein pattern based on the registered vein pattern registered within the vein pattern authentication apparatus.

**21.** A vein pattern registration method for registering a vein pattern acquired by radiating light to a portion of a living body, comprising the steps of:

capturing an image of a body surface of the portion of the living body with near-infrared light and generating near-infrared light imaging data;
extracting a vein pattern from the near-infrared light imaging data and generating a near-infrared light vein pattern;
capturing an image of the body surface with visible light and generating visible light imaging data;
extracting a vein pattern from the visible light imaging data and generating a visible light vein pattern;
comparing the near-infrared light vein pattern with the visible light vein pattern;
determining presence of a pseudo-vein pattern intentionally formed on a part of the captured body surface based on a comparison result; and
registering the near-infrared light vein pattern based on a determination result.

**22.** The vein pattern registration method according to claim 21, wherein the step of comparing the near-infrared light vein pattern with the visible light vein pattern includes the step of:

calculating a correlation coefficient between the near-infrared light vein pattern and the visible light vein pattern.

**23.** The vein pattern registration method according to claim 22, wherein the step of determining presence of a pseudo-vein pattern includes the steps of:

comparing the calculated correlation coefficient with a predetermined threshold value for determination;
determining that the pseudo-vein pattern is not present when the calculated correlation coefficient is less than the predetermined threshold value for determination; and
determining that the pseudo-vein pattern is present when the calculated correlation coefficient is equal to or greater than the predetermined threshold value for determination.

**24.** The vein pattern registration method according to claim 21, wherein the step of generating the near-infrared light vein pattern and the step of generating the visible light vein pattern includes the step of:

using a differential filter that outputs a large value for a pixel having a large difference between the pixel and its surrounding pixels for a plurality of pixels constituting the near-infrared light imaging data and the visible light imaging data, respectively.

**25.** The vein pattern registration method according to claim 24, wherein
the differential filter is a derivative filter.

**26.** The vein pattern registration method according to claim 25, wherein
the differential filter is a Laplacian of Gaussian (Log) filter.

**27.** A vein pattern authentication method for authenticating a vein pattern acquired by radiating light to a portion of a living body, comprising the steps of:

capturing an image of a body surface of the portion of the living body with near-infrared light and generating near-infrared light imaging data;
extracting a vein pattern from the near-infrared light imaging data and generating a near-infrared light vein pattern;
capturing an image of the body surface with visible light and generating visible light imaging data;
extracting a vein pattern from the visible light imaging data and generating a visible light vein pattern;
comparing the near-infrared light vein pattern with the visible light vein pattern;
determining presence of a pseudo-vein pattern intentionally formed on a part of the captured body surface based on a comparison result; and
comparing an already registered vein pattern with the near-infrared light vein pattern and authenticating the near-infrared light vein pattern based on a determination result.

**28.** The vein pattern authentication method according to claim 27, wherein the step of comparing the near-infrared light vein pattern with the visible light vein pattern includes the step of:

calculating a correlation coefficient between the near-infrared light vein pattern and the visible light vein pattern.

**29.** The vein pattern authentication method according to claim 28, wherein the step of determining presence of a pseudo-vein pattern includes the steps of:

comparing the calculated correlation coefficient with a predetermined threshold value for determination;
determining that the pseudo-vein pattern is not present when the calculated correlation coefficient is less than the predetermined threshold value for determination; and
determining that the pseudo-vein pattern is present when the calculated correlation coefficient is equal to or greater than the predetermined threshold value for determination.

**30.** The vein pattern authentication method according to claim 27, wherein the step of generating the near-infrared light vein pattern and the step of generating the visible light vein pattern includes the step of:

using a differential filter that outputs a large value for a pixel having a large difference between the pixel and its surrounding pixels for a plurality of pixels constituting the near-infrared light imaging data and the visible light imaging data, respectively.

**31.** The vein pattern authentication method according to claim 30, wherein the differential filter is a derivative filter.

**32.** The vein pattern authentication method according to claim 31, wherein the differential filter is a Laplacian of Gaussian (Log) filter.

**33.** A program for causing a computer controlling a vein pattern registration apparatus for registering a vein pattern acquired by radiating light to a portion of a living body to execute:

an imaging function for capturing images of a body surface of the portion of the living body with near-infrared light and visible light, and generating near-infrared light imaging data and visible light imaging data, respectively;
a vein pattern extraction function for extracting vein patterns from the near-infrared light imaging data and the visible light imaging data to generate a near-infrared light vein pattern and a visible light vein pattern, respectively;
a pseudo-vein pattern determination function for determining presence of a pseudo-vein pattern intentionally formed on a part of the captured body surface by comparing the near-infrared light vein pattern with the visible light vein pattern; and
a vein pattern registration function for registering the near-infrared light vein pattern based on a determination result to generate a registered vein pattern.

**34.** A program for causing a computer controlling a vein pattern authentication apparatus for authenticating a vein pattern acquired by radiating light to a portion of a living body to execute:

an imaging function for capturing images of a body surface of the portion of the living body with near-infrared light and visible light, and generating near-infrared light imaging data and visible light imaging data, respectively;
a vein pattern extraction function for extracting vein patterns from the near-infrared light imaging data and the visible light imaging data to generate a near-infrared light vein pattern and a visible light vein pattern, respectively;
a pseudo-vein pattern determination function for determining presence of a pseudo-vein pattern intentionally formed on a part of the captured body surface by comparing the near-infrared light vein pattern with the visible light vein pattern; and
a vein pattern authentication function for comparing an already registered vein pattern with the near-infrared light vein pattern and authenticating the near-infrared light vein pattern based on a determination result.

**35.** A vein data configuration comprising:

a vein data storage area containing data that correspond to a vein pattern of an individual and are to be verified with image data acquired by capturing an image of a body surface of a portion of a living body with near-infrared light; and
a correlation coefficient storage area containing a correlation coefficient between the image data acquired by capturing the image with the near-infrared light and image data acquired by capturing an image of the body surface with visible light.

**36.** The vein data configuration according to claim 35, wherein
the vein data configuration further includes a parameter storage area containing a parameter changing an output property of a differential filter outputting a high output for a pixel that differs largely from its surrounding pixels, for each pixel constituting the image data acquired by capturing the image with the near-infrared light, and
the parameter significantly changes an output value of the differential filter, when the image data acquired by capturing the image with the near-infrared light have a difference greater than a difference between a value indicating a vein portion and a value indicating a non-vein portion.

FIG. 1

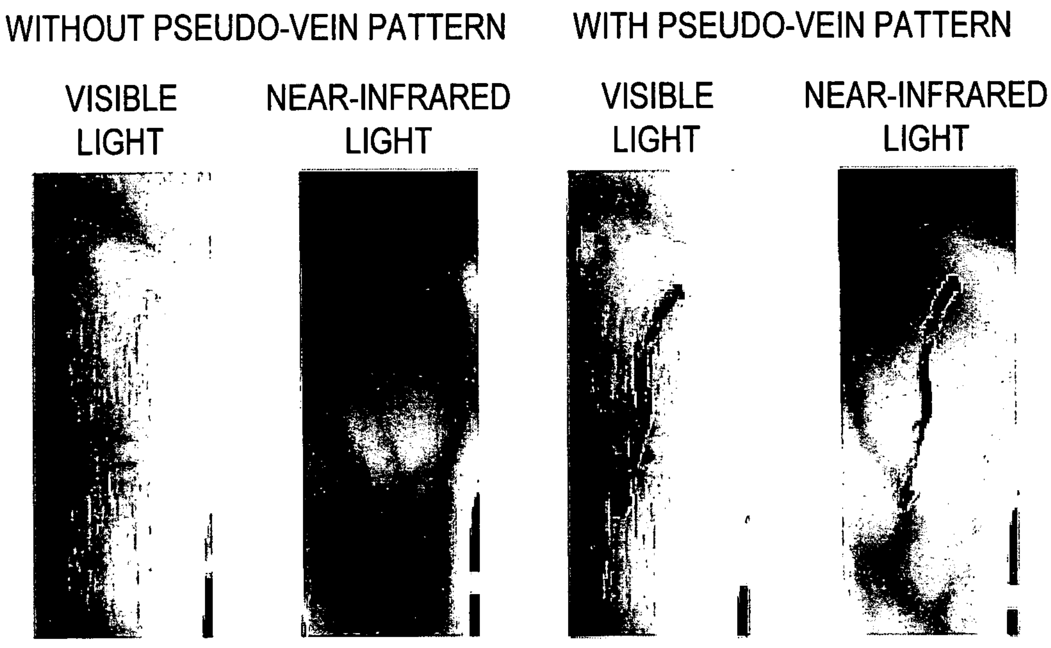
WITHOUT PSEUDO-VEIN PATTERN
WITH PSEUDO-VEIN PATTERN
VISIBLE LIGHT
NEAR-INFRARED LIGHT
VISIBLE LIGHT
NEAR-INFRARED LIGHT

# FIG. 2

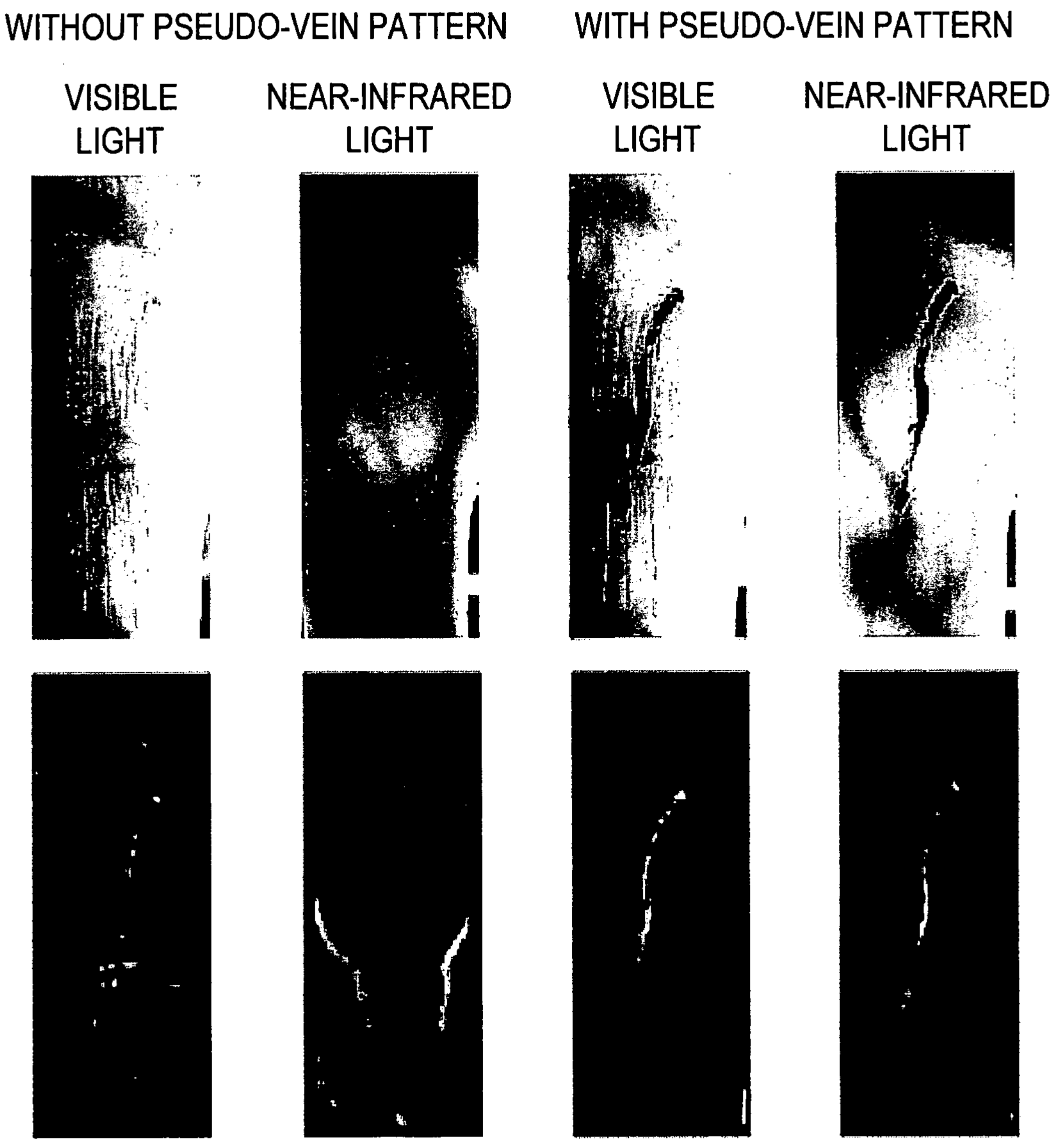
WITHOUT PSEUDO-VEIN PATTERN
WITH PSEUDO-VEIN PATTERN
VISIBLE LIGHT
NEAR-INFRARED LIGHT
VISIBLE LIGHT
NEAR-INFRARED LIGHT

FIG. 3

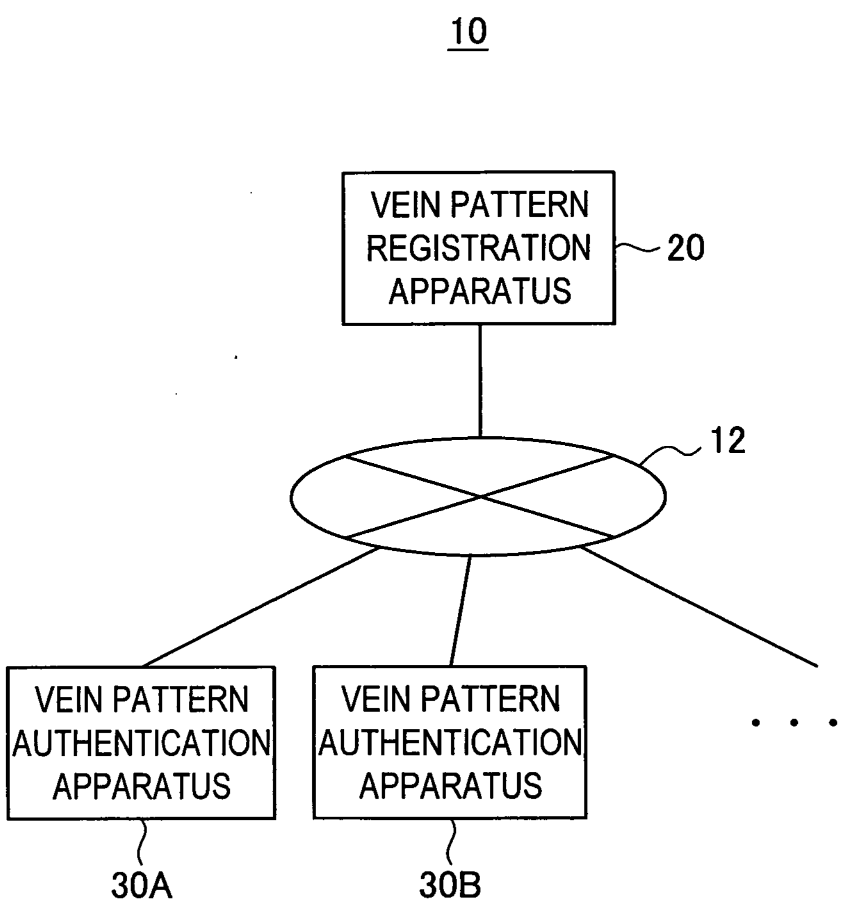
10
VEIN PATTERN REGISTRATION APPARATUS
20
12
VEIN PATTERN AUTHENTICATION APPARATUS
VEIN PATTERN AUTHENTICATION APPARATUS
30A
30B

FIG. 4

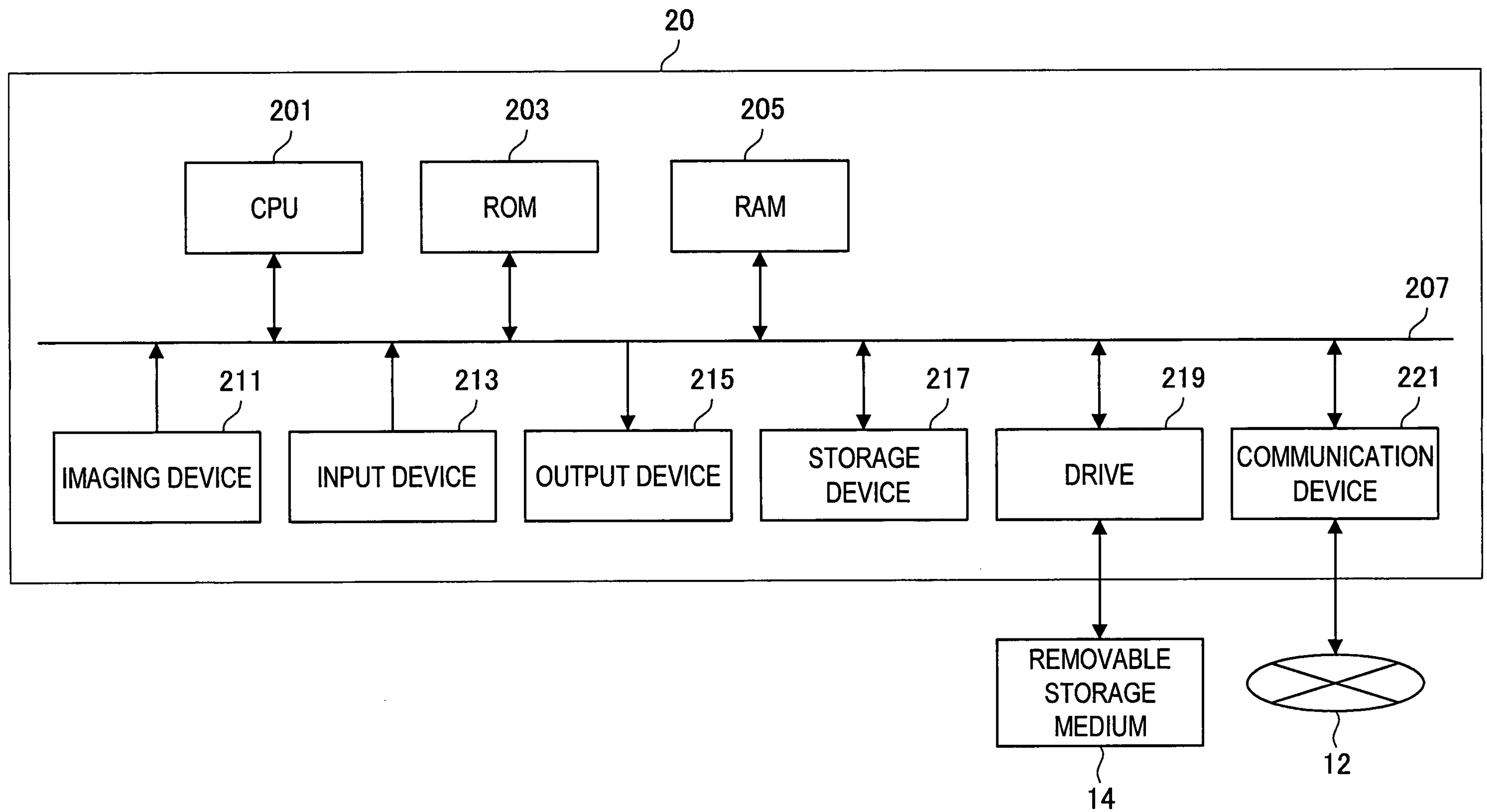
20
201
CPU
203
ROM
205
RAM
207
211
IMAGING DEVICE
213
INPUT DEVICE
215
OUTPUT DEVICE
217
STORAGE DEVICE
219
DRIVE
221
COMMUNICATION DEVICE
REMOVABLE STORAGE MEDIUM
14
12

FIG. 5

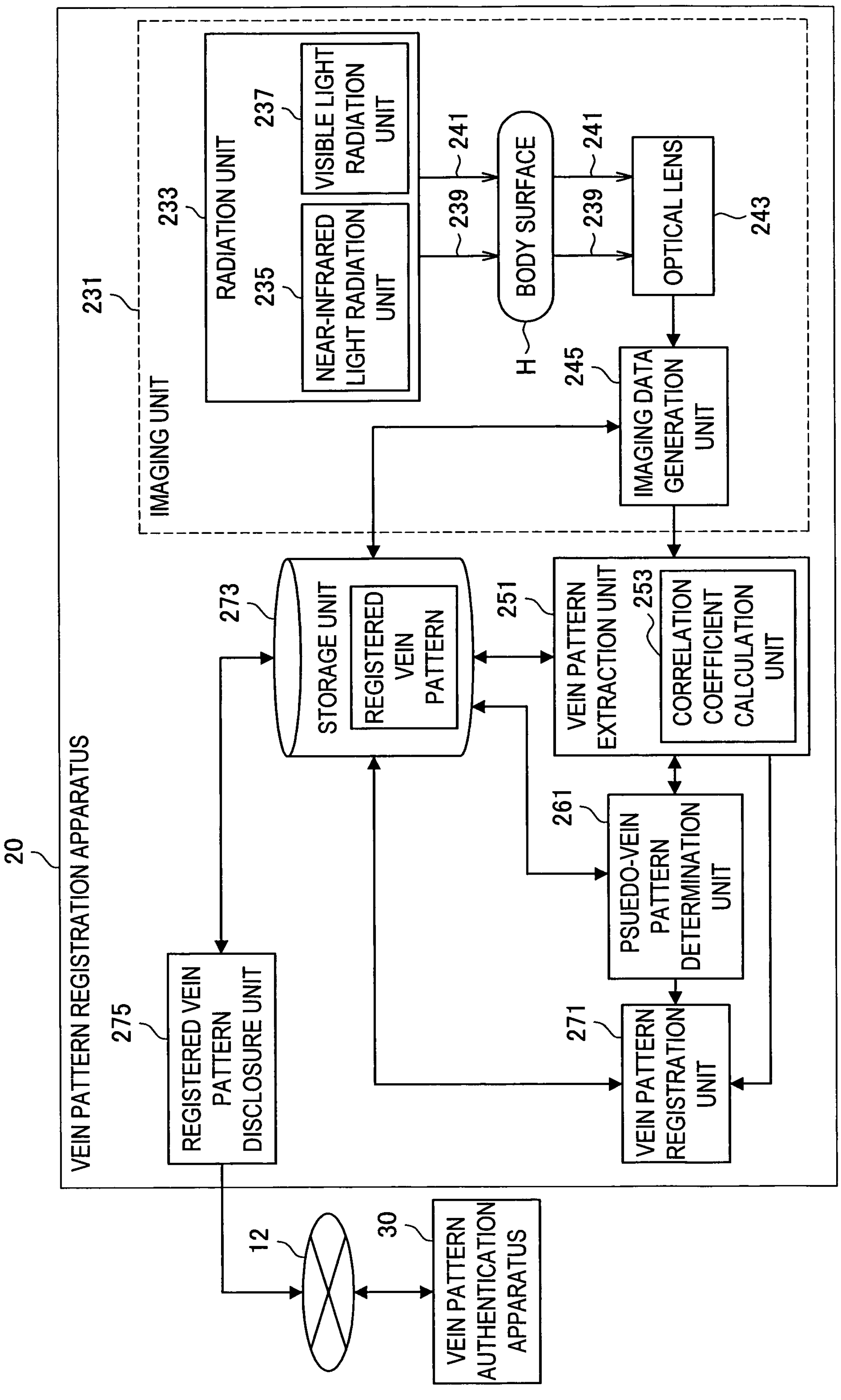
20
VEIN PATTERN REGISTRATION APPARATUS
231
IMAGING UNIT
233
RADIATION UNIT
235
NEAR-INFRARED LIGHT RADIATION UNIT
237
VISIBLE LIGHT RADIATION UNIT
239
241
H
BODY SURFACE
243
OPTICAL LENS
245
IMAGING DATA GENERATION UNIT
273
STORAGE UNIT
REGISTERED VEIN PATTERN
251
VEIN PATTERN EXTRACTION UNIT
253
CORRELATION COEFFICIENT CALCULATION UNIT
261
PSUEDO-VEIN PATTERN DETERMINATION UNIT
275
REGISTERED VEIN PATTERN DISCLOSURE UNIT
271
VEIN PATTERN REGISTRATION UNIT
12
30
VEIN PATTERN AUTHENTICATION APPARATUS

FIG. 6

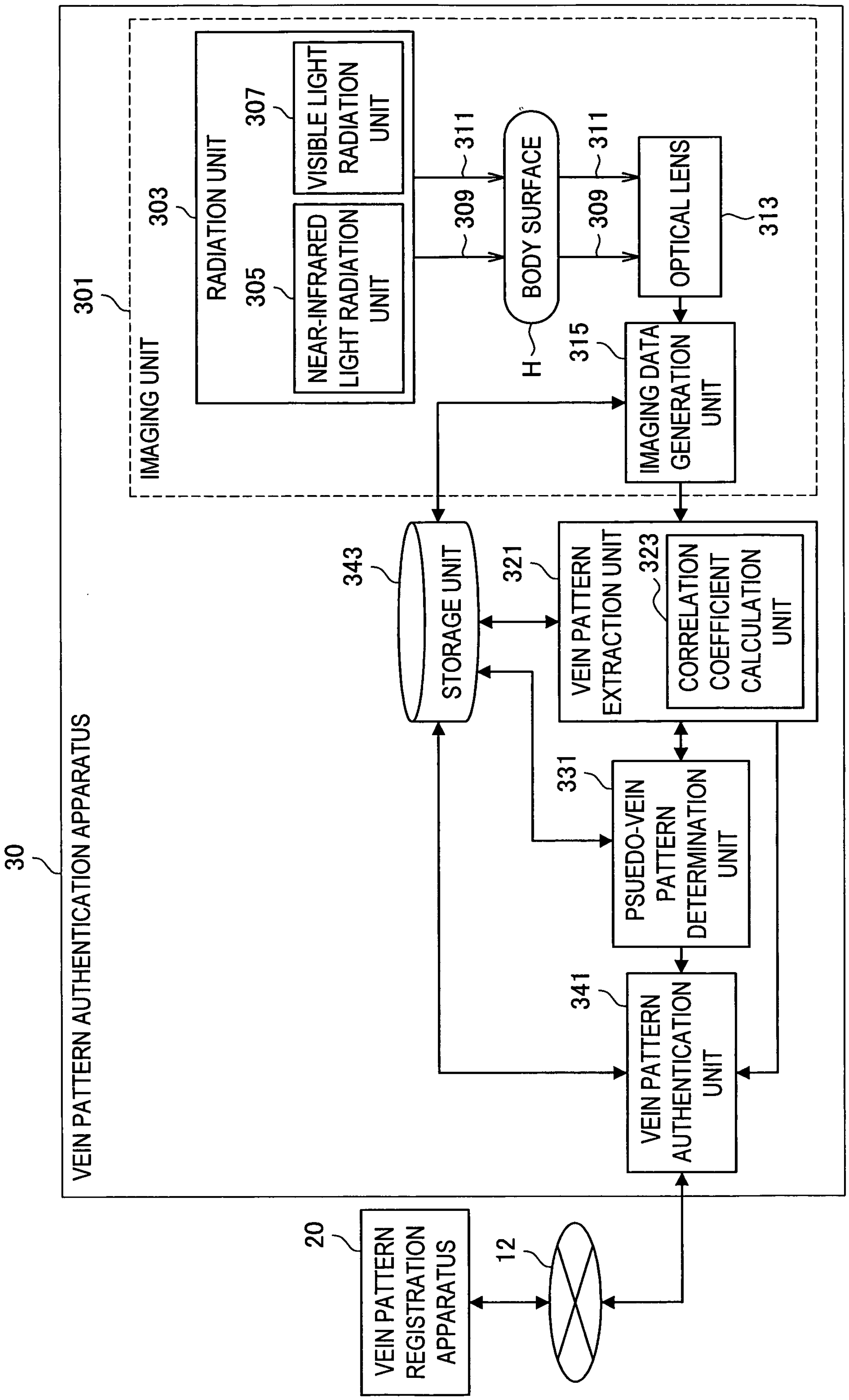
30
VEIN PATTERN AUTHENTICATION APPARATUS
301
IMAGING UNIT
303
RADIATION UNIT
305
NEAR-INFRARED LIGHT RADIATION UNIT
307
VISIBLE LIGHT RADIATION UNIT
309
311
H
BODY SURFACE
313
OPTICAL LENS
315
IMAGING DATA GENERATION UNIT
343
STORAGE UNIT
321
VEIN PATTERN EXTRACTION UNIT
323
CORRELATION COEFFICIENT CALCULATION UNIT
331
PSUEDO-VEIN PATTERN DETERMINATION UNIT
341
VEIN PATTERN AUTHENTICATION UNIT
12
20
VEIN PATTERN REGISTRATION APPARATUS

FIG. 7

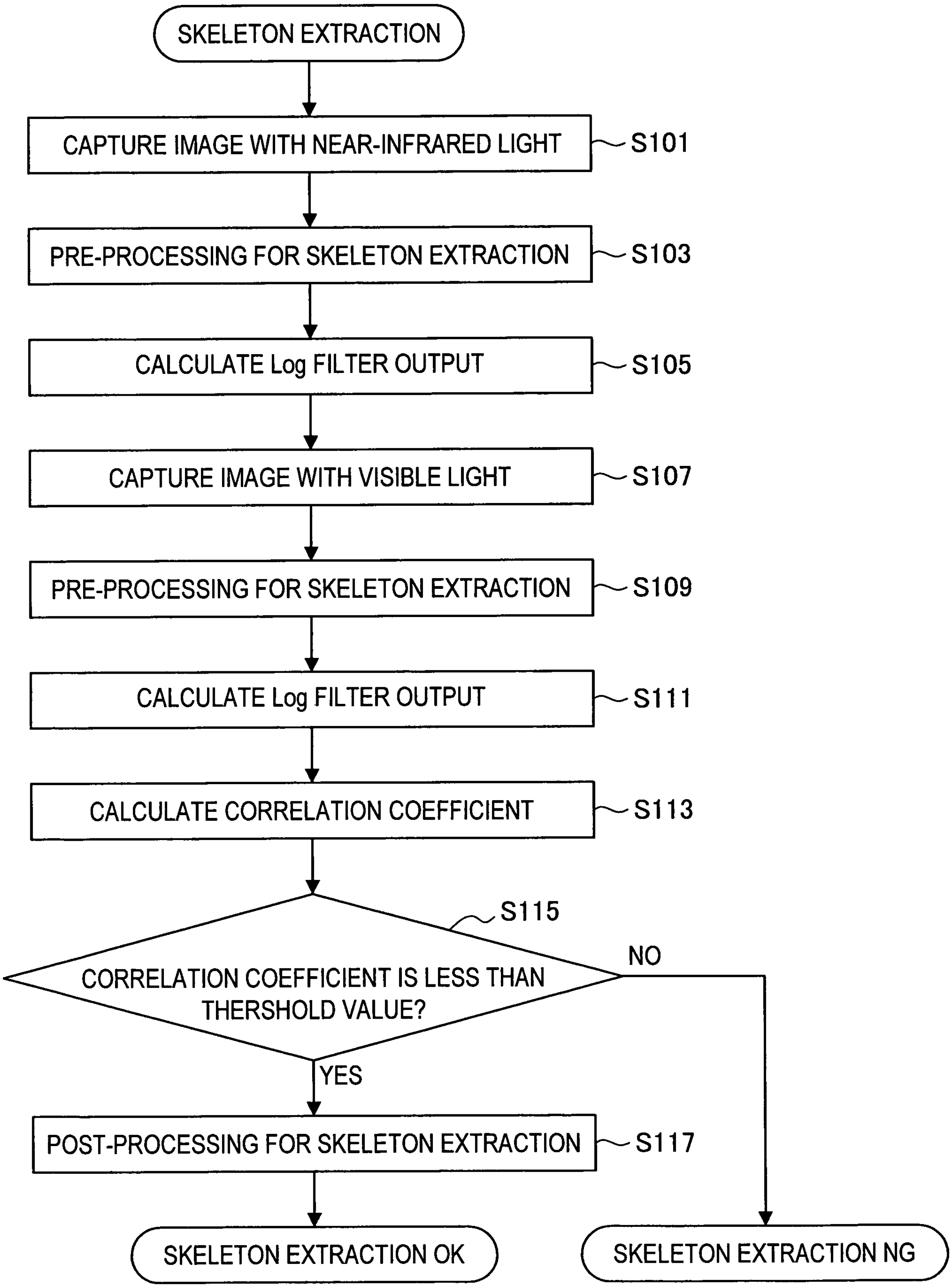
SKELETON EXTRACTION
CAPTURE IMAGE WITH NEAR-INFRARED LIGHT
S101
PRE-PROCESSING FOR SKELETON EXTRACTION
S103
CALCULATE Log FILTER OUTPUT
S105
CAPTURE IMAGE WITH VISIBLE LIGHT
S107
PRE-PROCESSING FOR SKELETON EXTRACTION
S109
CALCULATE Log FILTER OUTPUT
S111
CALCULATE CORRELATION COEFFICIENT
S113
S115
CORRELATION COEFFICIENT IS LESS THAN THERSHOLD VALUE?
NO
YES
POST-PROCESSING FOR SKELETON EXTRACTION
S117
SKELETON EXTRACTION OK
SKELETON EXTRACTION NG

# FIG. 8

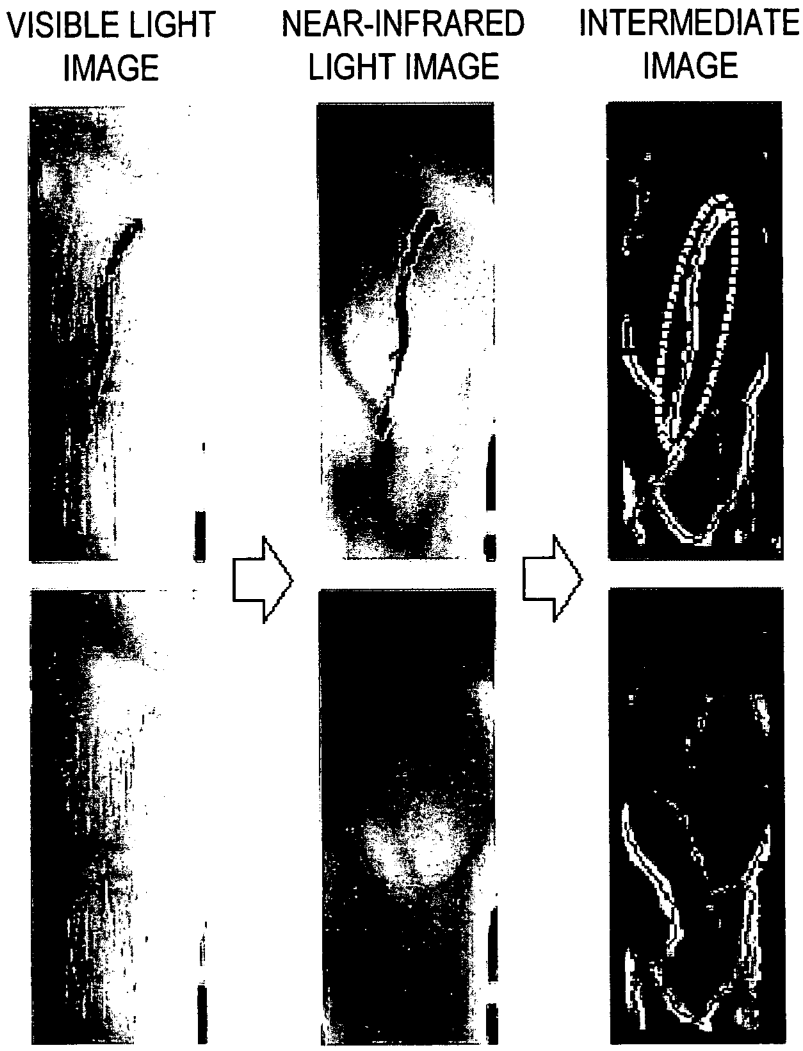
VISIBLE LIGHT IMAGE
NEAR-INFRARED LIGHT IMAGE
INTERMEDIATE IMAGE

**INTERNATIONAL SEARCH REPORT**

International application No.
PCT/JP2008/057950

A. CLASSIFICATION OF SUBJECT MATTER
*G06T1/00*(2006.01)i, *A61B5/117*(2006.01)i, *G06T7/00*(2006.01)i

According to International Patent Classification (IPC) or to both national classification and IPC

B. FIELDS SEARCHED

Minimum documentation searched (classification system followed by classification symbols)
G06T1/00, A61B5/117, G06T7/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br><br><br>Y | JP 2005-71118 A (Hitachi, Ltd.),<br>17 March, 2005 (17.03.05),<br>Par. Nos. [0016] to [0039]; Fig. 5<br>& US 2005/0047632 A1 & EP 1524621 A2<br>& DE 602004004469 D & HK 1070722 A<br>& CA 2472563 A1 | 1-3,7-9,<br>13-15,19-23,<br>27-29,33-36<br>4-6,10-12,<br>16-18,24-26,<br>30-32 |
| Y | JP 2005-56282 A (Sony Corp.),<br>03 March, 2005 (03.03.05),<br>Par. No. [0107]<br>(Family: none) | 4-6,10-12,<br>16-18,24-26,<br>30-32 |
| A | JP 2006-98340 A (Sharp Corp.),<br>13 April, 2006 (13.04.06),<br>Par. Nos. [0030] to [0033]; Fig. 3<br>(Family: none) | 1-36 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 May, 2008 (12.05.08) | 20 May, 2008 (20.05.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2008/057950

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-331239 A (Hitachi-Omron Terminal Solutions, Corp.), 07 December, 2006 (07.12.06), Par. No. [0015] (Family: none) | 1-36 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005259345 A

### Non-patent literature cited in the description

- **Tsutomu Matsumoto.** Biometric Authentication in Financial Transactions. *9th Study Group on Problem of Forged ATM Cards,* 15 April 2005